# EUROPEAN PATENT APPLICATION

(11) **EP 4 530 295 A2**
(43) Date of publication of application: **02.04.2025**
(21) Application number: 24198861.7
(22) Date of filing: 06.09.2024
(51) Int. Cl.: C07K 16/06, A61K 39/00, A61P 37/06

(54) **FCRN ANTAGONISTS AND METHODS OF USE**

(30) Priority: 07.09.2023 US 202363581179 P
(71) Applicant: argenx BV, 9052 Gent (BE)
(72) Inventor: Brinkhaus, Maximilian, 9052 Ghent (BE); Balbino, Bianca, 9052 Ghent (BE); Verheesen, Peter, 9052 Ghent (BE); Sips, Magdalena, 9052 Ghent (BE); Vidarsson, Gestur, 1066 CX Amsterdam (NL)
(74) Representative: Boult Wade Tennant LLP

(57) **Abstract**

Provided herein are human neonatal Fc receptor (FcRn) antagonists with increased affinity for Fc gamma receptors. Polynucleotides, vectors, host cells, and methods of production are also provided herein. Methods of treating an antibody-mediated disorder with an FcRn antagonist are further provided.

## Description

### FIELD

The present disclosure relates to human neonatal Fc receptor (FcRn) antagonists with enhanced FcyR binding affinity, compositions comprising these FcRn antagonists, and methods of reducing the level of serum IgG antibodies (e.g., autoantibodies) in a subject using these FcRn antagonists and compositions.

### BACKGROUND

Immunoglobulin gamma (IgG) antibodies play a key role in the pathology of many disorders, such as autoimmune diseases, inflammatory diseases, and disorders in which the pathology is characterized by over-expression of IgG antibodies.

The half-life of IgG in the serum is prolonged relative to the serum half-life of other plasma proteins due, in part, to the binding of the Fc region of IgG to the Fc receptor, FcRn. FcRn binds to IgG and protects the IgG from transport to degradative lysosomes by recycling it back to the extracellular compartment. This recycling is facilitated by the pH-dependent binding of IgG to FcRn, where the IgG/FcRn interaction is stronger at acidic endosomal pH than at extracellular physiological pH.

When the serum concentration of IgG reaches a level that exceeds available FcRn molecules, unbound IgG is not protected from lysosomal degradation and will consequently have a reduced serum half-life. Thus, inhibition of IgG binding to FcRn reduces the serum half-life of IgG by preventing endosomal recycling of IgG. Agents that antagonize the binding of IgG to FcRn, such as FcRn-binding molecules, are useful for regulating, treating, or preventing antibody-mediated disorders, such as autoimmune diseases or inflammatory diseases.

Efgartigimod is a modified human immunoglobulin (Ig) gamma (IgG) 1-derived Fc of the za allotype that binds with nanomolar affinity to human FcRn. Efgartigimod encompasses the IgG1 Fc-region and has been engineered using ABDEG technology to increase its affinity for FcRn at both physiological and acidic pH. The increased affinity for FcRn of efgartigimod at both acidic and physiological pH results in a blockage of FcRn-mediated recycling of IgGs. Efgartigimod has been approved as a weekly intravenous or subcutaneous injection for use in the treatment of generalized myasthenia gravis in the U.S., Europe, China, and Japan and is under development for the treatment of several other antibody-mediated disorders.

FcRn in endothelial cells was originally considered primarily responsible for IgG recycling. However, more recent studies have shown that bone-marrow-derived cells contribute about 50% of the IgG recycling capacity in vivo. Myeloid cells play a pivotal role in FcRn-mediated IgG recycling as evidenced by their strong IgG-degradative capacity when FcRn is lacking only in these cells. Evidence for cooperative mechanisms of Fc gamma receptors (FcyRs) and FcRn in the context of myeloid cell activation upon IgG immune complex binding and processing of IgG has been reported.

There is a need in the art for improved agents that antagonize FcRn binding to IgG, for use in the treatment of antibody-mediated disorders.

### SUMMARY

The instant disclosure is directed to novel neonatal Fc receptor (FcRn) antagonists with enhanced FcyR binding affinity. Also provided are pharmaceutical compositions comprising these FcRn antagonists, nucleic acids encoding these FcRn antagonists, expression vectors and host cells for making these FcRn antagonists, and methods of treating a subject using these FcRn antagonists. The FcRn antagonists provided herein are particularly advantageous because they enhance serum IgG depletion and therefore have utility in the treatment of antibody-mediated disorders, such as autoimmune diseases.

In an aspect, provided herein is an FcRn antagonist comprising a variant IgG Fc region, wherein the variant IgG Fc region comprises or consists of a first Fc domain and a second Fc domain which form a dimer, wherein: (a) at least one of the first Fc domain and the second Fc domain comprise amino acids K, F, and Y at EU positions 433, 434, and 436, respectively; and (b) at least one of the first Fc domain and the second Fc domain comprise: (i) amino acid A at EU position 236; and/or (ii) amino acids D, D, D, D, G, and R, at EU positions 233, 237, 238, 268, 271, and 330, respectively.

In another aspect, provided herein is an FcRn antagonist comprising a variant IgG Fc region, wherein the variant IgG Fc region comprises or consists of a first Fc domain and a second Fc domain which form a dimer, wherein: (a) at least one of the first Fc domain and the second Fc domain comprise amino acids Y, T, E, K, F, and Y at EU positions 252, 254, 256, 433, 434, and 436, respectively; and (b) at least one of the first Fc domain and the second Fc domain comprise: (i) amino acid A at EU position 236; and/or (ii) amino acids D, D, D, D, G, and R, at EU positions 233, 237, 238, 268, 271, and 330, respectively.

In some embodiments, the first Fc domain comprises amino acids Y, T, E, K, F, and Y at EU positions 252, 254, 256, 433, 434, and 436, respectively, and the second Fc domain comprises amino acid A at EU position 236; or the first Fc domain comprises amino acids A, Y, T, E, K, F, and Y at EU positions 236, 252, 254, 256, 433, 434, and 436, respectively, and the second Fc domain comprises amino acid A at EU position 236. In some embodiments, the second Fc domain further comprises amino acids K, F, and Y at EU positions 433, 434, and 436, respectively.

In some embodiments, both the first Fc domain and the second Fc domain comprise amino acids Y, T, E, K, F, and Y at EU positions 252, 254, 256, 433, 434, and 436, respectively. In some embodiments, both the first Fc domain and the second Fc domain comprise amino acid A at EU position 236.

In some embodiments, the FcRn antagonist comprises two, three, or four FcRn binding regions.

In some embodiments, the variant IgG Fc region binds to FcRn with a higher affinity at pH 6.0 as compared to a corresponding wild-type IgG Fc region. In some embodiments, the variant IgG Fc region binds to FcRn with a higher affinity at pH 7.4 as compared to a corresponding wild-type IgG Fc region. In some embodiments, the variant IgG Fc region binds to FcyRIIa with a higher affinity at pH 6.0 and/or at pH 7.4 as compared to a corresponding wild-type IgG Fc region.

In some embodiments, the first Fc domain and/or the second Fc domain is an IgG1 Fc domain. In some embodiments, the first Fc domain and/or the second Fc domain is a human IgG Fc domain. In some embodiments, the first Fc domain and/or the second Fc domain is a human IgG1 Fc domain. In some embodiments, both the first Fc domain and the second Fc domain are IgG1 Fc domains. In some embodiments, both the first Fc domain and the second Fc domain are human IgG Fc domains. In some embodiments, both the first Fc domain and the second Fc domain are human IgG1 Fc domains.

In some embodiments, the first Fc domain and/or the second Fc domain comprise or consist of an amino acid sequence independently selected from an amino acid sequence set forth in SEQ ID NOs: 7-9. In some embodiments, the first Fc domain and/or the second Fc domain comprise or consist of the amino acid sequence set forth in SEQ ID NO: 8. In some embodiments, both the first Fc domain and the second Fc domain comprise or consist of an amino acid sequence independently selected from an amino acid sequence set forth in SEQ ID NOs: 7-9. In some embodiments, both the first Fc domain and the second Fc domain comprise or consist of the amino acid sequence set forth in SEQ ID NO: 8.

In some embodiments, the first Fc domain and/or the second Fc domain comprise or consist of an amino acid sequence independently selected from an amino acid sequence set forth in SEQ ID NOs: 13-15. In some embodiments, the first Fc domain and/or the second Fc domain comprise or consist of the amino acid sequence set forth in SEQ ID NO: 14. In some embodiments, both the first Fc domain and the second Fc domain comprise or consist of an amino acid sequence independently selected from an amino acid sequence set forth in SEQ ID NOs: 13-15. In some embodiments, both the first Fc domain and the second Fc domain comprise or consist of the amino acid sequence set forth in SEQ ID NO: 14.

In some embodiments, the variant IgG Fc region is afucosylated.

In another aspect, provided herein is a polypeptide comprising an Fc domain comprising amino acids A, Y, T, E, K, F, and Y at EU positions 236, 252, 254, 256, 433, 434, and 436, respectively. In some embodiments, the polypeptide comprises or consists of an amino acid sequence selected from an amino acid sequence set forth in SEQ ID NOs: 7-9. In some embodiments, the polypeptide is afucosylated.

In another aspect, provided herein is a polypeptide comprising an Fc domain comprising amino acids D, D, D, Y, T, E, D, G, R, K, F, and Y at EU positions 233, 237, 238, 252, 254, 256, 268, 271, 330, 433, 434, and 436, respectively. In some embodiments, the polypeptide comprises or consists of an amino acid sequence selected from an amino acid sequence set forth in SEQ ID NOs: 13-15. In some embodiments, the polypeptide is afucosylated.

Also provided is a polynucleotide or polynucleotides encoding any FcRn antagonist or any polypeptide described herein.

Also provided is an expression vector comprising any polynucleotide or polynucleotides described herein.

Also provided is a host cell comprising any polynucleotide or polynucleotides described herein, or any expression vector described herein.

Also provided is a method for producing an FcRn antagonist, comprising culturing any host cell described herein under conditions which permit the expression of the FcRn antagonist.

Also provided is a pharmaceutical composition comprising any FcRn antagonist described herein, or any polypeptide described herein, and at least one pharmaceutically acceptable carrier.

Also provided is any FcRn antagonist, polypeptide, or pharmaceutical composition described herein for use as a medicament.

Also provided is a method of reducing serum levels of IgG in a subject comprising administering to the subject an effective amount of any FcRn antagonist, polypeptide, or pharmaceutical composition described herein.

Also provided is a method of enhancing intracellular uptake of IgG in myeloid cells in a subject comprising administering to the subject an effective amount of any FcRn antagonist, polypeptide, or pharmaceutical composition described herein.

Also provided is a method of treating an antibody-mediated disorder in a subject comprising administering to the subject an effective amount of any FcRn antagonist, polypeptide, or pharmaceutical composition described herein. In some embodiments, the antibody-mediated disorder is an IgG-mediated disorder. In some embodiments, the antibody-mediated disorder is an autoimmune disease. In some embodiments, the FcRn antagonist or polypeptide is administered to the subject simultaneously or sequentially with an additional therapeutic agent.

Also provided is any FcRn antagonist, polypeptide, or pharmaceutical composition described herein, for use in reducing serum levels of IgG, enhancing intracellular uptake of IgG in myeloid cells, or treating an antibody-mediated disorder, in a subject.

Also provided is any FcRn antagonist, or polypeptide, described herein, for the manufacture of a medicament for reducing serum levels of IgG, enhancing intracellular uptake of IgG in myeloid cells, or treating an antibody-mediated disorder, in a subject.

Also provided is the use of any FcRn antagonist, polypeptide, or pharmaceutical composition described herein, for the manufacture of a medicament for reducing serum levels of IgG, enhancing intracellular uptake of IgG in myeloid cells, or treating an antibody-mediated disorder, in a subject.

### BRIEF DESCRIPTION OF THE DRAWINGS

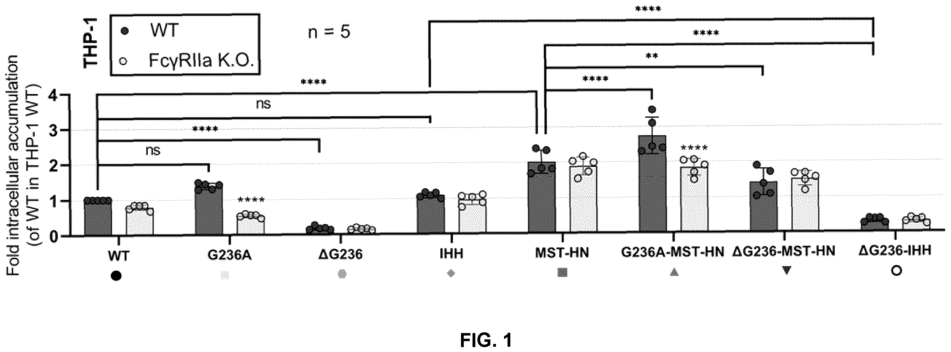

**FIG. 1** shows intracellular accumulation of Fc fragment variants in THP-1 and THP-1 FcyRIIa KO cells. Statistically significant differences are indicated by asterisks * < 0.05, ** < 0.01, **** <0.0001.

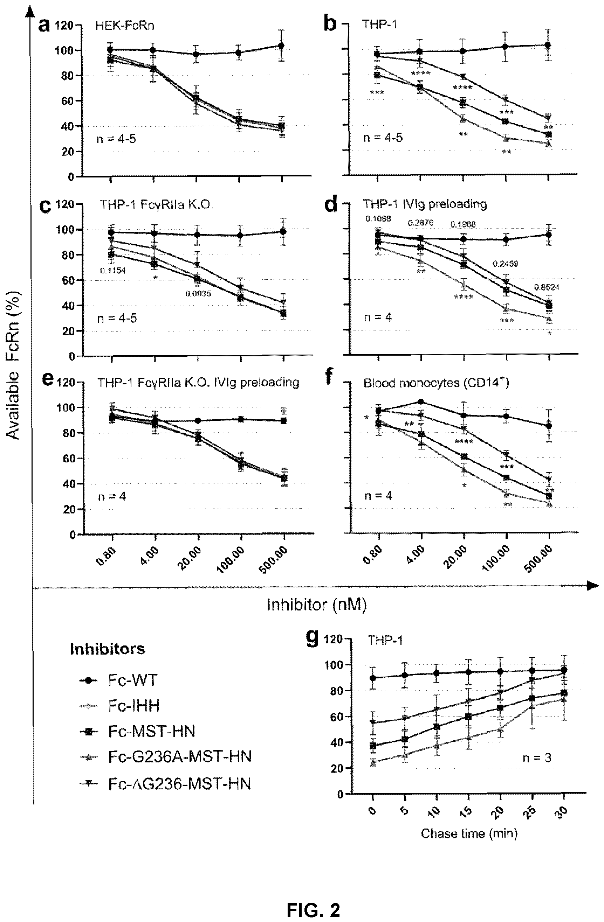

**FIG. 2** shows FcRn occupancy by Fc fragment variants plotted as percentage of medium control in FcRn overexpressing HEK-FcRn cells (panel a); THP-1 cells (panel b); THP-1 FcyRIIa knock out (K.O.) cells (panel c); THP-1 cells loaded with IVIg to block FcyRI (panel d); THP-1 FcyRIIa K.O. cells loaded with IVIg (panel e); blood monocytes (CD14+) (panel f); and THP-1 cells in a load and chase setup (panel g). Error bars indicate standard deviation. Statistically significant differences are indicated by asterisks * < 0.05, ** < 0.01, *** <0.001, **** <0.0001.

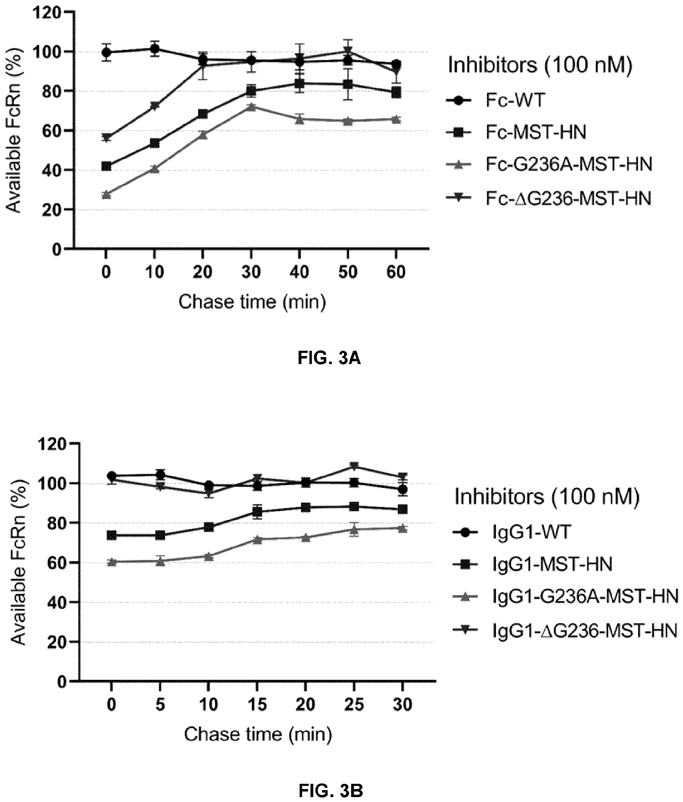

**FIGs. 3A-3B** shows FcRn occupancy by Fc fragment variants **(****FIG. 3A****)** and full-length anti-biotin IgG1 antibody variants **(****FIG. 3B****).** Error bars indicate standard deviation.

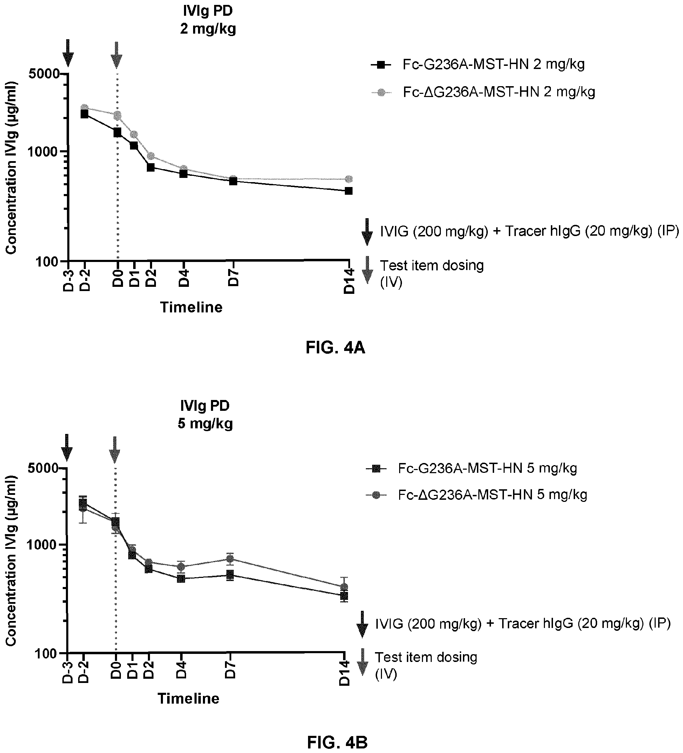

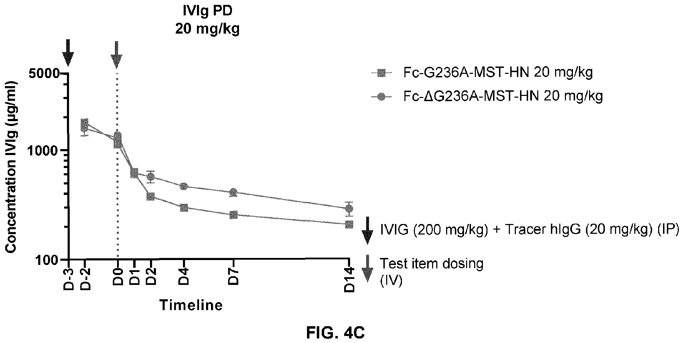

**FIGs. 4A-4C** show the effect of an Fc fragment with enhanced FcRn and FcyRIIa binding (Fc-G236A-MST-HN) or an Fc fragment with enhanced FcRn binding in which FcyR binding is eliminated (Fc-ΔG236-MST-HN) administered at 2 mg/kg **(****FIG. 4A****),** 5 mg/kg **(****FIG. 4B****),** and 20 mg/kg**(****FIG. 4C****)** on serum IVIg levels in humanized FcRn/FcyR mice.

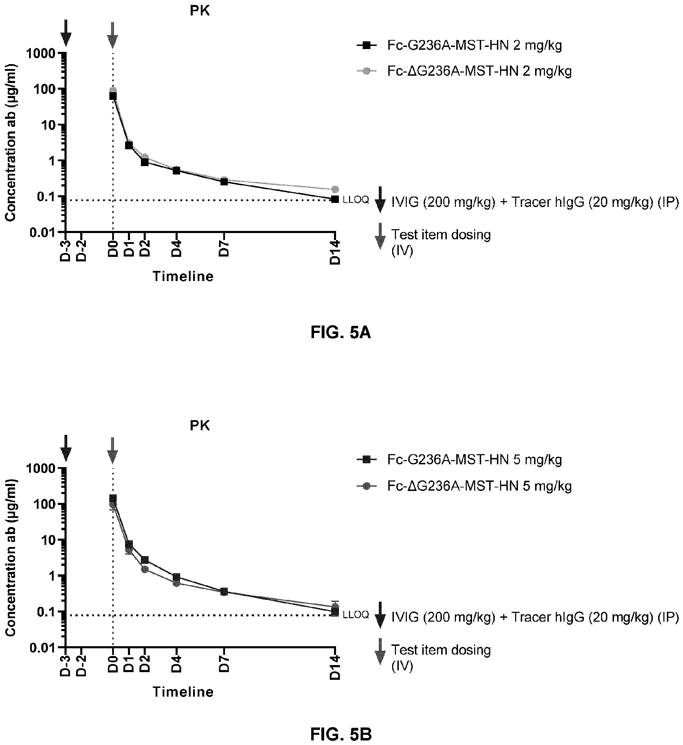

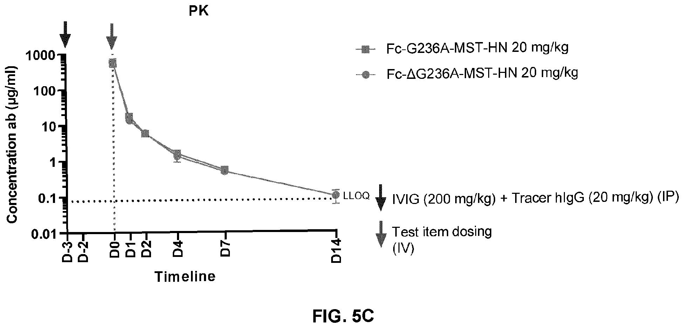

**FIGs. 5A-5C** show the pharmacokinetic profile of an Fc fragment with enhanced FcRn and FcyRIIa binding (Fc-G236A-MST-HN) or an Fc fragment with enhanced FcRn binding in which FcyR binding is eliminated (Fc-ΔG236-MST-HN) administered at 2 mg/kg **(****FIG. 5A****),** 5 mg/kg **(****FIG. 5B****),** and 20 mg/kg **(****FIG. 5C****)** to humanized FcRn/FcyR mice.

### DETAILED DESCRIPTION

The present disclosure provides FcRn antagonists with enhanced FcyR binding affinity. Nucleic acids encoding such FcRn antagonists, vectors, host cells, methods of manufacture, and methods for their use in treating antibody-mediated disorders are also provided herein.

### Definitions

As used herein, the term "FcRn" refers to a neonatal Fc receptor. Exemplary FcRn molecules include human FcRn encoded by the FCGRT gene as set forth in RefSeq NM 004107. The amino acid sequence of the corresponding protein is set forth in RefSeq NP_004098.

As used herein, the term "FcRn antagonist" refers to any agent that specifically binds to FcRn and inhibits the binding of immunoglobulin to FcRn (*e.g*., human FcRn). In an embodiment, the FcRn antagonist comprises an Fc region (*e.g.,* a variant Fc region disclosed herein) that specifically binds to FcRn through the Fc region and inhibits the binding of immunoglobulin to FcRn. In an embodiment, the FcRn antagonist is not a full-length IgG antibody. In an embodiment, the FcRn antagonist comprises an antigen-binding domain that binds a target antigen and a variant Fc region. In an embodiment, the term "FcRn antagonist" refers to an antibody or antigen-binding fragment thereof that specifically binds to FcRn via its antigen-binding domain and/or via its Fc region and inhibits the binding of the Fc region of immunoglobulin (*e.g*., IgG autoantibodies) to FcRn.

As used herein, the term "affinity" or "binding affinity" refers to the strength of the binding interaction between two molecules. As used herein, the term "equilibrium dissociation constant" or "K_{D}" refers to the propensity of bound complex of two molecules to dissociate into two free molecules. Thus, as the binding affinity increases, the K_{D} decreases.

As used herein, the term "specifically binds" refers to the ability of any molecule to preferentially bind with a given target. For example, a molecule that specifically binds to a given target can bind to other molecules, generally with lower affinity as determined by, *e.g.,* immunoassays, BIAcore^{™}, KinExA 3000 instrument (Sapidyne Instruments, Boise, Id.), or other assays known in the art. In a specific embodiment, molecules that specifically bind to a given target bind to the antigen with a K_{D} that is at least 2 logs, 2.5 logs, 3 logs, 4 logs or less than the K_{D} when the molecules bind non-specifically to another target.

The determination of "percent identity" between two sequences (*e.g*., amino acid sequences or nucleic acid sequences) can be accomplished using a mathematical algorithm. A specific, non-limiting example of a mathematical algorithm utilized for the comparison of two sequences is the algorithm of Karlin S & Altschul SF, (1990) PNAS 87: 2264-2268, modified as in Karlin S & Altschul SF, (1993) PNAS 90: 5873-5877, each of which is herein incorporated by reference in its entirety. Such an algorithm is incorporated into the NBLAST and XBLAST programs of Altschul SF et al., (1990) J Mol Biol 215: 403, which is herein incorporated by reference in its entirety. BLAST nucleotide searches can be performed with the NBLAST nucleotide program parameters set, *e.g.,* at score=100, wordlength=12 to obtain nucleotide sequences homologous to a nucleic acid molecule described herein. BLAST protein searches can be performed with the XBLAST program parameters set, *e.g.,* at score=50, wordlength=3 to obtain amino acid sequences homologous to a protein molecule described herein. To obtain gapped alignments for comparison purposes, Gapped BLAST can be utilized as described in Altschul SF et al., (1997) Nuc Acids Res 25: 3389-3402, which is herein incorporated by reference in its entirety. Alternatively, PSI BLAST can be used to perform an iterated search which detects distant relationships between molecules. *Id.* When utilizing BLAST, Gapped BLAST, and PSI BLAST programs, the default parameters of the respective programs (*e.g*., of XBLAST and NBLAST) can be used (*see, e.g.,* National Center for Biotechnology Information (NCBI) on the worldwide web, ncbi.nlm.nih.gov). Another specific, non-limiting example of a mathematical algorithm utilized for the comparison of sequences is the algorithm of Myers and Miller, (1988) CABIOS 4:11-17, which is herein incorporated by reference in its entirety. Such an algorithm is incorporated in the ALIGN program (version 2.0) which is part of the GCG sequence alignment software package. When utilizing the ALIGN program for comparing amino acid sequences, a PAM120 weight residue table, a gap length penalty of 12, and a gap penalty of 4 can be used.

The percent identity between two sequences can be determined using techniques similar to those described above, with or without allowing gaps. In calculating percent identity, typically only exact matches are counted.

As used herein, the terms "antibody" and "antibodies" include full-length antibodies, antigen-binding fragments of full-length antibodies, and molecules comprising antibody CDRs, VH domains (VH), or VL domains (VL). Examples of antibodies include monoclonal antibodies, recombinantly produced antibodies, monospecific antibodies, multi-specific antibodies (including bispecific antibodies), human antibodies, humanized antibodies, chimeric antibodies, immunoglobulins, synthetic antibodies, tetrameric antibodies comprising two heavy chain and two light chain molecules, an antibody light chain monomer, an antibody heavy chain monomer, an antibody light chain dimer, an antibody heavy chain dimer, an antibody light chain-antibody heavy chain pair, intrabodies, heteroconjugate antibodies, antibody-drug conjugates, single-domain antibodies (sdAb), monovalent antibodies, single chain antibodies or single-chain Fvs (scFv), camelid antibodies, affibody molecules, VHH fragments, Fab fragments, F(ab')₂ fragments, disulfide-linked Fvs (sdFv), anti-idiotypic (anti-Id) antibodies (including, *e.g.*, anti-anti-Id antibodies), and antigen-binding fragments of any of the above. Antibodies can be of any isotype (*e.g.,* IgG, IgE, IgM, IgD, IgA, or IgY), any subclass (*e.g.,* IgG₁, IgG₂, IgG₃, IgG₄, IgA₁, or IgA₂), or species (*e.g*., mouse IgG₂ₐ or IgG_{2b}) of immunoglobulin molecule.

As used herein, the term "antigen-binding domain" refers to any polypeptide that specifically binds to an antigen. Examples of antigen-binding domains include polypeptides derived from antibodies, such as Fab fragments, F(ab')₂ fragments, disulfide-linked Fvs (sdFv), single-chain Fvs (scFv), CDRs, VH domains (VH), VL domains (VL), single-domain antibodies (sdAb), VHH fragments, camelid antibodies, and antigen-binding fragments of any of the above. The term also encompasses synthetic antigen-binding proteins or antibody mimetic proteins such as, for example, anticalins and DARPins.

As used herein, the term "Fc region" refers to the portion of an immunoglobulin formed by the Fc domains of its two heavy chains. The Fc region can be a wild-type Fc region (native Fc region) or a variant Fc region. A native Fc region is homodimeric. The Fc region can be derived from any native immunoglobulin. In some embodiments, the Fc region is formed from an IgA, IgD, IgE, or IgG heavy chain constant region. In some embodiments, the Fc region is formed from an IgG heavy chain constant region. In some embodiments, the IgG heavy chain is an IgG₁, IgG2, IgG3, or IgG4 heavy chain constant region. In some embodiments, the Fc region is formed from an IgG1 heavy chain constant region. In some embodiments, the IgG1 heavy chain constant region comprises a G1m1(a), G1m2(x), G1m3(f), or G1m17(z) allotype. *See, e.g.*, Jefferis and Lefranc (2009) mAbs 1(4): 332-338, and de Taeye et al., (2020) Front Immunol. 11: 740, incorporated herein by reference in their entirety.

As used herein, the term "variant Fc region" refers to a variant of an Fc region with one or more alteration(s) relative to a native Fc region. Alterations can include amino acid substitutions, additions and/or deletions, linkage of additional moieties, and/or alteration of the native glycans. The term encompasses heterodimeric Fc regions where each of the constituent Fc domains is different. The term also encompasses single chain Fc regions where the constituent Fc domains are linked together by a linker moiety.

As used herein, the term "Fc domain" refers to the portion of a single immunoglobulin heavy chain comprising both the CH2 and CH3 domains of the antibody. In some embodiments, the Fc domain comprises at least a portion of a hinge (*e.g.,* upper, middle, and/or lower hinge region) region, a CH2 domain, and a CH3 domain. In some embodiments, the Fc domain does not include the hinge region.

As used herein, the term "hinge region" refers to the portion of a heavy chain molecule that joins the CH1 domain to the CH2 domain. In some embodiments, the hinge region is at most 70 amino acid residues in length. In some embodiments, this hinge region comprises approximately 11-17 amino acid residues and is flexible, thus allowing the two N-terminal antigen binding regions to move independently. In some embodiments, the hinge region is 12 amino acid residues in length. In some embodiments, the hinge region is 15 amino acid residues in length. In some embodiments, the hinge region is 62 amino acid residues in length. Hinge regions can be subdivided into three distinct domains: upper, middle, and lower hinge domains. The FcRn antagonists of the instant disclosure can include all or any portion of a hinge region. In some embodiments, the hinge region is from an IgG1 antibody. In some embodiments, the hinge region comprises the amino acid sequence of EPKSCDKTHTCPPCP (SEQ ID NO: 25).

As used herein, the term "EU position" refers to the amino acid position in the EU numbering convention for the Fc region described in Edelman, GM et al. Proc. Natl. Acad. USA, 63, 78-85 (1969) and Kabat et al., in "Sequences of Proteins of Immunological Interest," U.S. Dept. Health and Human Services, 5th edition, 1991.

As used herein, the term, "antibody-mediated disorder" refers to any disorder wherein the symptoms of the disorder are caused by abnormal levels of one or more antibodies in a subject. As used herein, the term "autoantibody-mediated disorder" refers to any disease or disorder in which the underlying pathology is caused, at least in part, by pathogenic IgG autoantibodies.

As used herein, the term "treat," "treating," and "treatment" refer to therapeutic or preventative measures described herein. The methods of "treatment" employ administration of a polypeptide to a subject having a disease or disorder, or predisposed to having such a disease or disorder, in order to prevent, cure, delay, reduce the severity of, or ameliorate one or more symptoms of the disease or disorder or recurring disease or disorder, or in order to prolong the survival of a subject beyond that expected in the absence of such treatment. In some embodiments, the methods of "treatment" employ administration of a polypeptide to a subject having a disease or disorder, or predisposed to having such a disease or disorder, in order to prevent, cure, delay, reduce the severity of, or ameliorate the disease or disorder or recurring disease or disorder.

As used herein, the term "effective amount" in the context of the administration of a therapy to a subject refers to the amount of a therapy that achieves a desired prophylactic or therapeutic effect.

As used herein, the term "dose" or "dosing" refers to an amount of an agent administered to a subject in a single administration.

As used herein, the term, "equivalent dose" refers to a dose of a first and a second therapeutic agent wherein the number of molecules of the first and second agents is about the same. In some embodiments, an equivalent dose is an equimolar dose. As used herein, the term "equimolar dose" refers to a dose of a first and a second therapeutic agent wherein the number of moles of the first and second agent is the same. In some embodiments, the first agent is an FcRn antagonist described herein, and the second agent is efgartigimod.

As used herein, the term "subject" includes any human or non-human animal. In an embodiment, the subject is a human or non-human mammal. In an embodiment, the subject is a human.

As used herein, the term "about" or "approximately" when referring to a measurable value, such as a dosage, encompasses variations of ±5% of a given value or range, as are appropriate to perform the methods disclosed herein.

### FcRn Antagonists

In one aspect, the disclosure provides novel FcRn antagonists. In general, these FcRn antagonists comprise a variant Fc region, or FcRn-binding fragment thereof, that binds specifically to FcRn with increased affinity and reduced pH dependence relative to a native (i.e., wild-type) Fc region and binds specifically to FcyR (e.g., FcyRIIa) with increased affinity relative to a native (i.e., wild-type) Fc region. These FcRn antagonists increase IgG internalization and clearance, leading to reduced serum levels of IgG.

FcRn antagonists disclosed herein include any molecule that binds to FcRn, including, but not limited to, any anti-FcRn antibody, any anti-FcRn binding region, or any Fc domain or Fc region.

In some embodiments, the FcRn antagonists disclosed herein comprise two, three, or four FcRn binding regions, such as an Fc region.

In some embodiments, the FcRn antagonists disclosed herein comprise one or more Fc regions, or FcRn binding fragment thereof, in combination with one or more antigen-binding domains (*e.g*., an sdAb, a Fab fragment, an scFv, or an antibody mimetic).

Any Fc region can be altered to produce a variant Fc region as disclosed herein. In general, an Fc region, or FcRn binding fragment thereof, is from a human immunoglobulin. It is understood, however, that the Fc region may be derived from an immunoglobulin of any other mammalian species, including for example, a camelid species, a rodent (*e.g.,* a mouse, rat, rabbit, guinea pig) or non-human primate (*e.g*., chimpanzee, macaque) species. Moreover, the Fc region or FcRn binding portion thereof may be derived from any immunoglobulin class, including IgM, IgG, IgD, IgA, and IgE, and any immunoglobulin isotype, including IgG₁, IgG2, IgG3, and IgG4. In an embodiment, the Fc region is an IgG Fc region (*e.g.,* a human IgG region). In an embodiment, the Fc region is an IgG1 Fc region (*e.g.,* a human IgG1 region). In an embodiment, the Fc region is a chimeric Fc region comprising portions of several different Fc regions. Suitable examples of chimeric Fc regions are set forth in US 2011/0243966A1, which is incorporated herein by reference in its entirety. A variety of Fc region gene sequences (*e.g*., human constant region gene sequences) are available in the form of publicly accessible deposits.

An Fc region can be further truncated or internally deleted to produce a minimal FcRn binding fragment thereof. The ability of an Fc-region fragment to bind to FcRn can be determined using any art recognized binding assay *e.g.*, ELISA.

To enhance the manufacturability of the FcRn antagonists disclosed herein, it is preferable that the constituent Fc regions do not comprise any non-disulfide bonded cysteine residues. Accordingly, in an embodiment, the Fc regions do not comprise a free cysteine residue.

In some embodiments, any Fc variant, or FcRn binding fragment thereof, that specifically binds to FcRn with increased affinity and reduced pH dependence relative to the native Fc region and specifically binds to FcyR (e.g., FcyRIIa) with increased affinity relative to the native Fc region can be used herein. In an embodiment, the variant Fc region comprises amino acid alterations, substitutions, insertions, and/or deletions that confer the desired characteristics. In some embodiments, the FcRn antagonist comprises a variant Fc region, or FcRn binding fragment thereof, which binds to FcRn with a higher affinity at pH 5.5 as compared to a corresponding wild-type Fc region. In some embodiments, the FcRn antagonist comprises a variant Fc region, or FcRn binding fragment thereof, which binds to FcRn with a higher affinity at pH 6.0 and/or at pH 7.4 as compared to a corresponding wild-type Fc region. In some embodiments, the FcRn antagonist comprises a variant Fc region, or FcRn binding fragment thereof, which binds to FcRn with a higher affinity at both acidic and neutral pH as compared to a corresponding wild-type Fc region.

In some embodiments, the FcRn antagonist comprises a variant Fc region, or FcRn binding fragment thereof, which binds to FcyR with a higher affinity compared to a corresponding wild-type Fc region. In some embodiments, the FcRn antagonist comprises a variant Fc region, or FcRn binding fragment thereof, which binds to FcyR with a higher affinity at pH 6.0 and/or at pH 7.4 as compared to a corresponding wild-type Fc region.

In some embodiments, the FcRn antagonist comprises a variant Fc region, or FcRn binding fragment thereof, which binds to one or more of FcyRIIa, FcyRIIb, and FcyRIII with a higher affinity compared to a corresponding wild-type Fc region. In some embodiments, the FcRn antagonist comprises a variant Fc region, or FcRn binding fragment thereof, which binds to one or more of FcyRIIa, FcyRIIb, and FcyRIII with a higher affinity at pH 6.0 and/or at pH 7.4 as compared to a corresponding wild-type Fc region.

In some embodiments, the FcRn antagonist comprises a variant Fc region, or FcRn binding fragment thereof, which binds to FcyRIIa with a higher affinity compared to a corresponding wild-type Fc region. In some embodiments, the FcRn antagonist comprises a variant Fc region, or FcRn binding fragment thereof, which binds to FcyRIIa with a higher affinity at pH 6.0 and/or at pH 7.4 as compared to a corresponding wild-type Fc region.

In some embodiments, the FcRn antagonist comprises a variant Fc region, or FcRn binding fragment thereof, which binds to FcyRIIb with a higher affinity compared to a corresponding wild-type Fc region. In some embodiments, the FcRn antagonist comprises a variant Fc region, or FcRn binding fragment thereof, which binds to FcyRIIb with a higher affinity at pH 6.0 and/or at pH 7.4 as compared to a corresponding wild-type Fc region.

In some embodiments, the FcRn antagonist comprises a variant Fc region, or FcRn binding fragment thereof, which binds to FcyRIII with a higher affinity compared to a corresponding wild-type Fc region. In some embodiments, the FcRn antagonist comprises a variant Fc region, or FcRn binding fragment thereof, which binds to FcyRIII with a higher affinity at pH 6.0 and/or at pH 7.4 as compared to a corresponding wild-type Fc region.

In some embodiments, the FcRn antagonist comprises a variant Fc region, or FcRn binding fragment thereof, which binds to two or more of FcyRIIa, FcyRIIb, and FcyRIII with a higher affinity compared to a corresponding wild-type Fc region. In some embodiments, the FcRn antagonist comprises a variant Fc region, or FcRn binding fragment thereof, which binds to two or more of FcyRIIa, FcyRIIb, and FcyRIII with a higher affinity at pH 6.0 and/or at pH 7.4 as compared to a corresponding wild-type Fc region.

In some embodiments, the FcRn antagonist comprises a variant Fc region, or FcRn binding fragment thereof, which binds to FcyRIIa, FcyRIIb, and FcyRIII with a higher affinity compared to a corresponding wild-type Fc region. In some embodiments, the FcRn antagonist comprises a variant Fc region, or FcRn binding fragment thereof, which binds to FcyRIIa, FcyRIIb, and FcyRIII with a higher affinity at pH 6.0 and/or at pH 7.4 as compared to a corresponding wild-type Fc region.

In some embodiments, the variant Fc region is derived from the Fc region of any native immunoglobulin. In some embodiments, the native immunoglobulin is a human immunoglobulin. In some embodiments, the immunoglobulin is IgA, IgD, IgE, or IgG. In some embodiments, the immunoglobulin is IgG. In some embodiments, the immunoglobulin is human IgA, human IgD, human IgE, or human IgG. In some embodiments, the immunoglobulin is human IgG. In some embodiments, the IgG is IgG1, IgG2, IgG3, or IgG4. In some embodiments, the human IgG is human IgG1, human IgG2, human IgG3, or human IgG4. In some embodiments, the variant Fc region varies from the human IgG1 Fc region. In some embodiments, the human IgG1 Fc region comprises a G1m1(a), G1m2(x), G1m3(f), or G1m17(z) allotype.

In some embodiments, the variant Fc region, or FcRn binding fragment thereof comprises or consists of at least one Fc domain. In some embodiments, the variant Fc region comprises or consists of two Fc domains. In some embodiments, the Fc domains are the same. In some embodiments, the Fc domains are different.

In certain embodiments, at least one of the variant Fc domains or FcRn binding fragments described herein comprises at least 1, 2, 3, 4, 5, 6, or 7 amino acids selected from the following: 233D; 236A; 237D; 237M; 238A; 238D; 239K; 248I; 250A; 250F; 250I; 250M; 250Q; 250S; 250V; 250W; 250Y; 252F; 252W; 252Y; 254T; 255E; 256D; 256E; 256Q; 257A; 257G; 257I; 257L; 257M; 257N; 257S; 257T; 257V; 258H; 265A; 268D; 270F; 271G; 286A; 286E; 289H; 297A; 298G; 303A; 305A; 307A; 307D; 307F; 307G; 307H; 307I; 307K; 307L; 307M; 307N; 307P; 307Q; 307R; 307S; 307V; 307W; 307Y; 308A; 308F; 308I; 308L; 308M; 308P; 308Q; 308T; 309A; 309D; 309E; 309P; 309R; 311A; 311H; 311I; 312A; 312H; 314K; 314R; 315A; 315H; 317A; 325G; 330R; 332V; 334L; 360H; 376A; 378V; 380A; 382A; 384A; 385D; 385H; 386P; 387E; 389A; 389S; 424A; 428A; 428D; 428F; 428G; 428H; 428I; 428K; 428L; 428N; 428P; 428Q; 428S; 428T; 428V; 428W; 428Y; 433K; 434A; 434F; 434H; 434S; 434W; 434Y; 436H; 436I; 436F; and 436Y, wherein the positions are defined in accordance with EU numbering. EU numbering refers to the convention for the Fc region described in Edelman, G.M. et al., Proc. Natl. Acad. Sci. USA, 63: 78-85 (1969); and Kabat et al., in "Sequences of Proteins of Immunological Interest", U.S. Dept. Health and Human Services, 5th edition, 1991, and wherein any combinations are contemplated.

In some embodiments, at least one of the variant Fc domains or FcRn binding fragments described herein comprises at least 1, 2, 3, 4, 5, 6, or 7 amino acids selected from the following: 233D; 236A; 237D; 238D; 252Y; 254T; 256E; 268D; 271G; 330R; 433K; 434F; 436F; and 436Y, wherein the positions are defined in accordance with EU numbering, and wherein any combinations are contemplated.

In an embodiment, the variant Fc region, or FcRn binding fragment thereof comprises or consists of two Fc domains. In an embodiment, the variant Fc region, or FcRn binding fragment thereof, comprises at least one Fc domain comprising amino acids K and F at EU positions 433, and 434, respectively. In an embodiment, the variant Fc region, or FcRn binding fragment thereof, comprises at least one Fc domain comprising amino acids K, F, and Y at EU positions 433, 434, and 436, respectively. In an embodiment, the variant Fc region, or FcRn binding fragment thereof, comprises at least one Fc domain comprising amino acids K, F, and F at EU positions 433, 434, and 436, respectively. In an embodiment, the variant Fc region, or FcRn binding fragment thereof consists of two Fc domains, both of which comprise amino acids K and F at EU positions 433 and 434, respectively. In an embodiment, the variant Fc region, or FcRn binding fragment thereof, consists of two Fc domains, both of which comprise amino acids K, F, and Y at EU positions 433, 434, and 436, respectively. In an embodiment, the variant Fc region, or FcRn binding fragment thereof, consists of two Fc domains, both of which comprise amino acids K, F, and F at EU positions 433, 434, and 436, respectively.

In an embodiment, the variant Fc region, or FcRn binding fragment thereof, comprises at least one Fc domain comprising amino acids Y, T, E, K, and F at EU positions 252, 254, 256, 433, and 434, respectively. In an embodiment, the variant Fc region, or FcRn binding fragment thereof, comprises at least one Fc domain comprising amino acids Y, T, E, K, F, and Y at EU positions 252, 254, 256, 433, 434, and 436, respectively. In an embodiment, the variant Fc region, or FcRn binding fragment thereof, comprises at least one Fc domain comprising amino acids Y, T, E, K, F, and F at EU positions 252, 254, 256, 433, 434, and 436, respectively. In an embodiment, the variant Fc region, or FcRn binding fragment thereof, comprises one Fc domain comprising amino acids Y, T, E, K, and F at EU positions 252, 254, 256, 433, and 434, respectively, and a second Fc domain comprising amino acid K and F at EU positions 433 and 434, respectively. In an embodiment, the variant Fc region, or FcRn binding fragment thereof, comprises one Fc domain comprising amino acids Y, T, E, K, F, and Y at EU positions 252, 254, 256, 433, 434, and 436, respectively, and a second Fc domain comprising amino acid K and F at EU positions 433 and 434, respectively. In an embodiment, the variant Fc region, or FcRn binding fragment thereof, comprises one Fc domain comprising amino acids Y, T, E, K, F, and F at EU positions 252, 254, 256, 433, 434, and 436, respectively, and a second Fc domain comprising amino acid K and F at EU positions 433 and 434, respectively. In an embodiment, the variant Fc region, or FcRn binding fragment thereof consists of two Fc domains, both of which comprise amino acids Y, T, E, K, and F at EU positions 252, 254, 256, 433, and 434, respectively. In an embodiment, the variant Fc region, or FcRn binding fragment thereof, consists of two Fc domains, both of which comprise amino acids Y, T, E, K, F, and Y at EU positions 252, 254, 256, 433, 434, and 436, respectively. In an embodiment, the variant Fc region, or FcRn binding fragment thereof, consists of two Fc domains, both of which comprise amino acids Y, T, E, K, F, and F at EU positions 252, 254, 256, 433, 434, and 436, respectively.

In an embodiment, the variant Fc region, or FcRn binding fragment thereof, comprises at least one Fc domain comprising at least one amino acid selected from the following: 233D, 236A, 237D, 238D, 268D, 271G, or 330R, wherein the positions are defined in accordance with EU numbering, and wherein any combinations are contemplated. In an embodiment, the variant Fc region, or FcRn binding fragment thereof, comprises at least one Fc domain comprising amino acid A at EU position 236. In an embodiment, the variant Fc region, or FcRn binding fragment thereof, comprises at least one Fc domain comprising amino acids D, D, D, D, G, and R. at EU positions 233, 237, 238, 268, 271, and 330, respectively.

In an embodiment, the variant Fc region, or FcRn binding fragment thereof, comprises at least one Fc domain comprising amino acids K and F at EU positions 433 and 434, respectively, and at least one Fc domain comprises amino acid A at EU position 236. In some embodiments, amino acids K and F at EU positions 433 and 434, respectively are present in one Fc domain, and amino acid A at EU position 236 is present in another Fc domain. In some embodiments, amino acids K and F at EU positions 433 and 434, respectively, and amino acid A at EU position 236 is present in the same Fc domain. In an embodiment, the variant Fc region, or FcRn binding fragment thereof, comprises at least one Fc domain comprising amino acids K, F, and Y at EU positions 433, 434, and 436, respectively, and at least one Fc domain comprises amino acid A at EU position 236. In some embodiments, amino acids K, F, and Y at EU positions 433, 434, and 436, respectively are present in one Fc domain, and amino acid A at EU position 236 is present in another Fc domain. In some embodiments, amino acids K, F, and Y at EU positions 433, 434, and 436, respectively, and amino acid A at EU position 236 is present in the same Fc domain. In an embodiment, the variant Fc region, or FcRn binding fragment thereof, comprises at least one Fc domain comprising amino acids K, F, and F at EU positions 433, 434, and 436, respectively, and at least one Fc domain comprises amino acid A at EU position 236. In some embodiments, amino acids K, F, and F at EU positions 433, 434, and 436, respectively are present in one Fc domain, and amino acid A at EU position 236 is present in another Fc domain. In some embodiments, amino acids K, F, and F at EU positions 433, 434, and 436, respectively, and amino acid A at EU position 236 is present in the same Fc domain.

In an embodiment, the variant Fc region, or FcRn binding fragment thereof, comprises at least one Fc domain comprising amino acids Y, T, E, K, and F at EU positions 252, 254, 256, 433, and 434, respectively, and at least one Fc domain comprises amino acid A at EU position 236. In some embodiments, amino acids Y, T, E, K, and F at EU positions 252, 254, 256, 433, and 434, respectively are present in one Fc domain, and amino acid A at EU position 236 is present in another Fc domain. In some embodiments, amino acids Y, T, E, K, and F at EU positions 252, 254, 256, 433, and 434, respectively, and amino acid A at EU position 236 is present in the same Fc domain. In an embodiment, the variant Fc region, or FcRn binding fragment thereof, comprises at least one Fc domain comprising amino acids Y, T, E, K, F, and Y at EU positions 252, 254, 256, 433, 434, and 436, respectively, and at least one Fc domain comprises amino acid A at EU position 236. In some embodiments, amino acids Y, T, E, K, F, and Y at EU positions 252, 254, 256, 433, 434, and 436, respectively are present in one Fc domain, and amino acid A at EU position 236 is present in another Fc domain. In some embodiments, amino acids Y, T, E, K, F, and Y at EU positions 252, 254, 256, 433, 434, and 436, respectively, and amino acid A at EU position 236 is present in the same Fc domain. In an embodiment, the variant Fc region, or FcRn binding fragment thereof, comprises at least one Fc domain comprising amino acids Y, T, E, K, F, and F at EU positions 252, 254, 256, 433, 434, and 436, respectively, and at least one Fc domain comprises amino acid A at EU position 236. In some embodiments, amino acids Y, T, E, K, F, and F at EU positions 252, 254, 256, 433, 434, and 436, respectively are present in one Fc domain, and amino acid A at EU position 236 is present in another Fc domain. In some embodiments, amino acids Y, T, E, K, F, and F at EU positions 252, 254, 256, 433, 434, and 436, respectively, and amino acid A at EU position 236 is present in the same Fc domain.

In an embodiment, the variant Fc region, or FcRn binding fragment thereof, comprises at least one Fc domain comprising amino acids K and F at EU positions 433 and 434, respectively, and at least one Fc domain comprises amino acids D, D, D, D, G, and R. at EU positions 233, 237, 238, 268, 271, and 330, respectively. In some embodiments, amino acids K and F at EU positions 433 and 434, respectively are present in one Fc domain, and amino acids D, D, D, D, G, and R, at EU positions 233, 237, 238, 268, 271, and 330, respectively are present in another Fc domain. In some embodiments, amino acids K and F at EU positions 433 and 434, respectively, and amino acids D, D, D, D, G, and R, at EU positions 233, 237, 238, 268, 271, and 330, respectively are present in the same Fc domain. In an embodiment, the variant Fc region, or FcRn binding fragment thereof, comprises at least one Fc domain comprising amino acids K, F, and Y at EU positions 433, 434, and 436, respectively, and at least one Fc domain comprises amino acids D, D, D, D, G, and R, at EU positions 233, 237, 238, 268, 271, and 330, respectively. In some embodiments, amino acids K, F, and Y at EU positions 433, 434, and 436, respectively are present in one Fc domain, and amino acids D, D, D, D, G, and R, at EU positions 233, 237, 238, 268, 271, and 330, respectively are present in another Fc domain. In some embodiments, amino acids K, F, and Y at EU positions 433, 434, and 436, respectively, and amino acids D, D, D, D, G, and R, at EU positions 233, 237, 238, 268, 271, and 330, respectively are present in the same Fc domain. In an embodiment, the variant Fc region, or FcRn binding fragment thereof, comprises at least one Fc domain comprising amino acids K, F, and F at EU positions 433, 434, and 436, respectively, and at least one Fc domain comprises amino acids D, D, D, D, G, and R, at EU positions 233, 237, 238, 268, 271, and 330, respectively. In some embodiments, amino acids K, F, and F at EU positions 433, 434, and 436, respectively are present in one Fc domain, and amino acids D, D, D, D, G, and R, at EU positions 233, 237, 238, 268, 271, and 330, respectively are present in another Fc domain. In some embodiments, amino acids K, F, and F at EU positions 433, 434, and 436, respectively, and amino acids D, D, D, D, G, and R, at EU positions 233, 237, 238, 268, 271, and 330, respectively are present in the same Fc domain.

In an embodiment, the variant Fc region, or FcRn binding fragment thereof, comprises at least one Fc domain comprising amino acids Y, T, E, K, and F at EU positions 252, 254, 256, 433, and 434, respectively, and at least one Fc domain comprises amino acids D, D, D, D, G, and R, at EU positions 233, 237, 238, 268, 271, and 330, respectively. In some embodiments, amino acids Y, T, E, K, and F at EU positions 252, 254, 256, 433, and 434, respectively are present in one Fc domain, and amino acids D, D, D, D, G, and R, at EU positions 233, 237, 238, 268, 271, and 330, respectively are present in another Fc domain. In some embodiments, amino acids Y, T, E, K, and F at EU positions 252, 254, 256, 433, and 434, respectively, and amino acids D, D, D, D, G, and R, at EU positions 233, 237, 238, 268, 271, and 330, respectively are present in the same Fc domain. In an embodiment, the variant Fc region, or FcRn binding fragment thereof, comprises at least one Fc domain comprising amino acids Y, T, E, K, F, and Y at EU positions 252, 254, 256, 433, 434, and 436, respectively, and at least one Fc domain comprises amino acids D, D, D, D, G, and R, at EU positions 233, 237, 238, 268, 271, and 330, respectively. In some embodiments, amino acids Y, T, E, K, F, and Y at EU positions 252, 254, 256, 433, 434, and 436, respectively are present in one Fc domain, and amino acids D, D, D, D, G, and R, at EU positions 233, 237, 238, 268, 271, and 330, respectively are present in another Fc domain. In some embodiments, amino acids Y, T, E, K, F, and Y at EU positions 252, 254, 256, 433, 434, and 436, respectively, and amino acids D, D, D, D, G, and R, at EU positions 233, 237, 238, 268, 271, and 330, respectively are present in the same Fc domain. In an embodiment, the variant Fc region, or FcRn binding fragment thereof, comprises at least one Fc domain comprising amino acids Y, T, E, K, F, and F at EU positions 252, 254, 256, 433, 434, and 436, respectively, and at least one Fc domain comprises amino acids D, D, D, D, G, and R, at EU positions 233, 237, 238, 268, 271, and 330, respectively. In some embodiments, amino acids Y, T, E, K, F, and F at EU positions 252, 254, 256, 433, 434, and 436, respectively are present in one Fc domain, and amino acids D, D, D, D, G, and R, at EU positions 233, 237, 238, 268, 271, and 330, respectively are present in another Fc domain. In some embodiments, amino acids Y, T, E, K, F, and F at EU positions 252, 254, 256, 433, 434, and 436, respectively, and amino acids D, D, D, D, G, and R, at EU positions 233, 237, 238, 268, 271, and 330, respectively are present in the same Fc domain.

In certain embodiments, at least one of the variant Fc domains or FcRn binding fragments described herein comprises a combination of amino acids selected from the following:
(i) K and F at EU positions 433 and 434, respectively;
(ii) K, F, and Y at EU positions 433, 434, and 436, respectively;
(iii) K, F, and F at EU positions 433, 434, and 436, respectively;
(iv) Y, T, E, K, and F at EU positions 252, 254, 256, 433, and 434, respectively;
(v) Y, T, E, K, F, and Y at EU positions 252, 254, 256, 433, 434, and 436, respectively; or
(vi) Y, T, E, K, F, and F at EU positions 252, 254, 256, 433, 434, and 436, respectively, and a combination of amino acids selected from the following;
(v) A at EU position 236; or
(vi) D, D, D, D, G, and R, at EU positions 233, 237, 238, 268, 271, and 330, respectively.

In an embodiment, one, two, or more mutations (*e.g*., amino acid substitutions) are introduced into the hinge region of a polypeptide described herein, such that the number of cysteine residues in the hinge region is altered (*e.g.,* increased or decreased) as described in, *e.g.*, U.S. Patent No. 5,677,425, herein incorporated by reference in its entirety. The number of cysteine residues in the hinge region may be altered to, *e.g.*, facilitate assembly of the light and heavy chains, or to alter (*e.g*., increase or decrease) the stability of the polypeptide.

In an embodiment, any of the constant region mutations or modifications described herein can be introduced into one or both heavy chain constant regions of a polypeptide described herein having two heavy chain constant regions. In an embodiment, any of the constant region mutations or modifications described herein can be introduced into the heavy chain constant region of a polypeptide described herein having one heavy chain constant region.

In an embodiment, any of the variant Fc regions described herein are afucosylated.

In an embodiment, the instant disclosure provides a polypeptide comprising one, two, three, or four binding sites for human FcRn, that specifically binds to FcRn and functions as an antagonist.

In certain embodiments, the variant Fc region is a heterodimer, where the constituent Fc domains are different from each other. Methods of producing Fc heterodimers are known in the art (*see, e.g.*, US 8,216,805, which is incorporated by reference herein in its entirety). In an embodiment, the variant Fc region is modified to promote heterodimerization. Such modifications are known in the art and any suitable means to promote heterodimerization may be used to generate the FcRn antagonists described herein. In some embodiments, the variant Fc region comprises one or more mutations of amino acid residues forming the interface of the CH3 domain of the Fc domains. In some embodiments, the variant Fc region comprises knob-into-hole mutations (*see, e.g.*, Intl. Publ. WO 2006/028936, incorporated by reference in its entirety). The mispairing of Ig heavy chains is reduced in this technology by mutating selected amino acids forming the interface of the CH3 domains in IgG. At positions within the CH3 domain at which the two heavy chains interact directly, one or more amino acids with a small side chain (hole) is/are introduced into the sequence of one heavy chain and one or more amino acids with a large side chain (knob) into the counterpart interacting residue location(s) on the other heavy chain. The Fc domains of an Fc region can be composed of immunoglobulin chains of the same subclass (*e.g*., IgG1 or IgG3) or different subclasses (*e.g*., IgG1 and IgG3, or IgG3 and IgG4).

In an embodiment, the FcRn antagonist consists of a variant Fc region, wherein the variant Fc region consists of two Fc domains which form a heterodimer, wherein the amino acid sequence of each of the Fc domains is independently selected from an amino acid sequence set forth in SEQ ID NOs: 1-24.

In an embodiment, the FcRn antagonist consists of or comprises a variant Fc region, wherein the variant Fc region consists of or comprises two Fc domains which form a heterodimer, wherein the first Fc domain consists of or comprises an amino acid sequence selected from an amino acid sequence set forth in SEQ ID NOs: 1-3, and the second Fc domain consists of or comprises an amino acid sequence selected from an amino acid sequence set forth in SEQ ID NOs: 4-15 or 19-24. In an embodiment, the FcRn antagonist consists of or comprises a variant Fc region, wherein the variant Fc region consists of or comprises two Fc domains which form a heterodimer, wherein the first Fc domain consists of or comprises an amino acid sequence selected from an amino acid sequence set forth in SEQ ID NOs: 1-3, and the second Fc domain consists of or comprises an amino acid sequence selected from an amino acid sequence set forth in SEQ ID NOs: 4-6. In an embodiment, the FcRn antagonist consists of or comprises a variant Fc region, wherein the variant Fc region consists of or comprises two Fc domains which form a heterodimer, wherein the first Fc domain consists of or comprises an amino acid sequence selected from an amino acid sequence set forth in SEQ ID NOs: 1-3, and the second Fc domain consists of or comprises an amino acid sequence selected from an amino acid sequence set forth in SEQ ID NOs: 7-9. In an embodiment, the FcRn antagonist consists of or comprises a variant Fc region, wherein the variant Fc region consists of or comprises two Fc domains which form a heterodimer, wherein the first Fc domain consists of or comprises an amino acid sequence selected from an amino acid sequence set forth in SEQ ID NOs: 1-3, and the second Fc domain consists of or comprises an amino acid sequence selected from an amino acid sequence set forth in SEQ ID NOs: 10-12. In an embodiment, the FcRn antagonist consists of or comprises a variant Fc region, wherein the variant Fc region consists of or comprises two Fc domains which form a heterodimer, wherein the first Fc domain consists of or comprises an amino acid sequence selected from an amino acid sequence set forth in SEQ ID NOs: 1-3, and the second Fc domain consists of or comprises an amino acid sequence selected from an amino acid sequence set forth in SEQ ID NOs: 13-15. In an embodiment, the FcRn antagonist consists of or comprises a variant Fc region, wherein the variant Fc region consists of or comprises two Fc domains which form a heterodimer, wherein the first Fc domain consists of or comprises an amino acid sequence selected from an amino acid sequence set forth in SEQ ID NOs: 1-3, and the second Fc domain consists of or comprises an amino acid sequence selected from an amino acid sequence set forth in SEQ ID NOs: 19-21. In an embodiment, the FcRn antagonist consists of or comprises a variant Fc region, wherein the variant Fc region consists of or comprises two Fc domains which form a heterodimer, wherein the first Fc domain consists of or comprises an amino acid sequence selected from an amino acid sequence set forth in SEQ ID NOs: 1-3, and the second Fc domain consists of or comprises an amino acid sequence selected from an amino acid sequence set forth in SEQ ID NOs: 22-24.

In an embodiment, the FcRn antagonist consists of or comprises a variant Fc region, wherein the variant Fc region consists of or comprises two Fc domains which form a heterodimer, wherein the first Fc domain consists of or comprises the amino acid sequence set forth in SEQ ID NO: 2, and the second Fc domain consists of or comprises an amino acid sequence selected from an amino acid sequence set forth in SEQ ID NOs: 4-15 or 19-24. In an embodiment, the FcRn antagonist consists of or comprises a variant Fc region, wherein the variant Fc region consists of or comprises two Fc domains which form a heterodimer, wherein the first Fc domain consists of or comprises the amino acid sequence set forth in SEQ ID NO: 2, and the second Fc domain consists of or comprises the amino acid sequence set forth in SEQ ID NO: 5. In an embodiment, the FcRn antagonist consists of or comprises a variant Fc region, wherein the variant Fc region consists of or comprises two Fc domains which form a heterodimer, wherein the first Fc domain consists of or comprises the amino acid sequence set forth in SEQ ID NO: 2, and the second Fc domain consists of or comprises the amino acid sequence set forth in SEQ ID NO: 8. In an embodiment, the FcRn antagonist consists of or comprises a variant Fc region, wherein the variant Fc region consists of or comprises two Fc domains which form a heterodimer, wherein the first Fc domain consists of or comprises the amino acid sequence set forth in SEQ ID NO: 2, and the second Fc domain consists of or comprises the amino acid sequence set forth in SEQ ID NO: 11. In an embodiment, the FcRn antagonist consists of or comprises a variant Fc region, wherein the variant Fc region consists of or comprises two Fc domains which form a heterodimer, wherein the first Fc domain consists of or comprises the amino acid sequence set forth in SEQ ID NO: 2, and the second Fc domain consists of or comprises the amino acid sequence set forth in SEQ ID NO: 14. In an embodiment, the FcRn antagonist consists of or comprises a variant Fc region, wherein the variant Fc region consists of or comprises two Fc domains which form a heterodimer, wherein the first Fc domain consists of or comprises the amino acid sequence set forth in SEQ ID NO: 2, and the second Fc domain consists of or comprises the amino acid sequence set forth in SEQ ID NO: 20. In an embodiment, the FcRn antagonist consists of or comprises a variant Fc region, wherein the variant Fc region consists of or comprises two Fc domains which form a heterodimer, wherein the first Fc domain consists of or comprises the amino acid sequence set forth in SEQ ID NO: 2, and the second Fc domain consists of or comprises the amino acid sequence set forth in SEQ ID NO: 23.

In an embodiment, the FcRn antagonist consists of or comprises a variant Fc region, wherein the variant Fc region consists of or comprises two Fc domains which form a heterodimer, wherein the first Fc domain consists of or comprises an amino acid sequence selected from an amino acid sequence set forth in SEQ ID NOs: 4-6, and the second Fc domain consists of or comprises an amino acid sequence selected from an amino acid sequence set forth in SEQ ID NOs: 7-9. In an embodiment, the FcRn antagonist consists of or comprises a variant Fc region, wherein the variant Fc region consists of or comprises two Fc domains which form a heterodimer, wherein the first Fc domain consists of or comprises an amino acid sequence selected from an amino acid sequence set forth in SEQ ID NOs: 4-6, and the second Fc domain consists of or comprises an amino acid sequence selected from an amino acid sequence set forth in SEQ ID NOs: 13-15. In an embodiment, the FcRn antagonist consists of or comprises a variant Fc region, wherein the variant Fc region consists of or comprises two Fc domains which form a heterodimer, wherein the first Fc domain consists of or comprises an amino acid sequence selected from an amino acid sequence set forth in SEQ ID NOs: 4-6, and the second Fc domain consists of or comprises an amino acid sequence selected from an amino acid sequence set forth in SEQ ID NOs: 19-21. In an embodiment, the FcRn antagonist consists of or comprises a variant Fc region, wherein the variant Fc region consists of or comprises two Fc domains which form a heterodimer, wherein the first Fc domain consists of or comprises an amino acid sequence selected from an amino acid sequence set forth in SEQ ID NOs: 4-6, and the second Fc domain consists of or comprises an amino acid sequence selected from an amino acid sequence set forth in SEQ ID NOs: 22-24.

In an embodiment, the FcRn antagonist consists of or comprises a variant Fc region, wherein the variant Fc region consists of or comprises two Fc domains which form a heterodimer, wherein the first Fc domain consists of or comprises the amino acid sequence set forth in SEQ ID NO: 5, and the second Fc domain consists of or comprises the amino acid sequence set forth in SEQ ID NO: 8. In an embodiment, the FcRn antagonist consists of or comprises a variant Fc region, wherein the variant Fc region consists of or comprises two Fc domains which form a heterodimer, wherein the first Fc domain consists of or comprises the amino acid sequence set forth in SEQ ID NO: 5, and the second Fc domain consists of or comprises the amino acid sequence set forth in SEQ ID NO: 14. In an embodiment, the FcRn antagonist consists of or comprises a variant Fc region, wherein the variant Fc region consists of or comprises two Fc domains which form a heterodimer, wherein the first Fc domain consists of or comprises the amino acid sequence set forth in SEQ ID NO: 5, and the second Fc domain consists of or comprises the amino acid sequence set forth in SEQ ID NO: 20. In an embodiment, the FcRn antagonist consists of or comprises a variant Fc region, wherein the variant Fc region consists of or comprises two Fc domains which form a heterodimer, wherein the first Fc domain consists of or comprises the amino acid sequence set forth in SEQ ID NO: 5, and the second Fc domain consists of or comprises the amino acid sequence set forth in SEQ ID NO: 23.

In an embodiment, the FcRn antagonist consists of or comprises a variant Fc region, wherein the variant Fc region consists of or comprises two Fc domains which form a heterodimer, wherein the first Fc domain consists of or comprises an amino acid sequence selected from an amino acid sequence set forth in SEQ ID NOs: 16-18, and the second Fc domain consists of or comprises an amino acid sequence selected from an amino acid sequence set forth in SEQ ID NOs: 4-15 or 19-24. In an embodiment, the FcRn antagonist consists of or comprises a variant Fc region, wherein the variant Fc region consists of or comprises two Fc domains which form a heterodimer, wherein the first Fc domain consists of or comprises an amino acid sequence selected from an amino acid sequence set forth in SEQ ID NOs: 16-18, and the second Fc domain consists of or comprises an amino acid sequence selected from an amino acid sequence set forth in SEQ ID NOs: 4-6. In an embodiment, the FcRn antagonist consists of or comprises a variant Fc region, wherein the variant Fc region consists of or comprises two Fc domains which form a heterodimer, wherein the first Fc domain consists of or comprises an amino acid sequence selected from an amino acid sequence set forth in SEQ ID NOs: 16-18, and the second Fc domain consists of or comprises an amino acid sequence selected from an amino acid sequence set forth in SEQ ID NOs: 7-9. In an embodiment, the FcRn antagonist consists of or comprises a variant Fc region, wherein the variant Fc region consists of or comprises two Fc domains which form a heterodimer, wherein the first Fc domain consists of or comprises an amino acid sequence selected from an amino acid sequence set forth in SEQ ID NOs: 16-18, and the second Fc domain consists of or comprises an amino acid sequence selected from an amino acid sequence set forth in SEQ ID NOs: 10-12. In an embodiment, the FcRn antagonist consists of or comprises a variant Fc region, wherein the variant Fc region consists of or comprises two Fc domains which form a heterodimer, wherein the first Fc domain consists of or comprises an amino acid sequence selected from an amino acid sequence set forth in SEQ ID NOs: 16-18, and the second Fc domain consists of or comprises an amino acid sequence selected from an amino acid sequence set forth in SEQ ID NOs: 13-15. In an embodiment, the FcRn antagonist consists of or comprises a variant Fc region, wherein the variant Fc region consists of or comprises two Fc domains which form a heterodimer, wherein the first Fc domain consists of or comprises an amino acid sequence selected from an amino acid sequence set forth in SEQ ID NOs: 16-18, and the second Fc domain consists of or comprises an amino acid sequence selected from an amino acid sequence set forth in SEQ ID NOs: 19-21. In an embodiment, the FcRn antagonist consists of or comprises a variant Fc region, wherein the variant Fc region consists of or comprises two Fc domains which form a heterodimer, wherein the first Fc domain consists of or comprises an amino acid sequence selected from an amino acid sequence set forth in SEQ ID NOs: 16-18, and the second Fc domain consists of or comprises an amino acid sequence selected from an amino acid sequence set forth in SEQ ID NOs: 22-24.

In an embodiment, the FcRn antagonist consists of or comprises a variant Fc region, wherein the variant Fc region consists of or comprises two Fc domains which form a heterodimer, wherein the first Fc domain consists of or comprises the amino acid sequence set forth in SEQ ID NO: 17, and the second Fc domain consists of or comprises an amino acid sequence selected from an amino acid sequence set forth in SEQ ID NOs: 4-15 or 19-24. In an embodiment, the FcRn antagonist consists of or comprises a variant Fc region, wherein the variant Fc region consists of or comprises two Fc domains which form a heterodimer, wherein the first Fc domain consists of or comprises the amino acid sequence set forth in SEQ ID NO: 17, and the second Fc domain consists of or comprises the amino acid sequence set forth in SEQ ID NO: 5. In an embodiment, the FcRn antagonist consists of or comprises a variant Fc region, wherein the variant Fc region consists of or comprises two Fc domains which form a heterodimer, wherein the first Fc domain consists of or comprises the amino acid sequence set forth in SEQ ID NO: 17, and the second Fc domain consists of or comprises the amino acid sequence set forth in SEQ ID NO: 8. In an embodiment, the FcRn antagonist consists of or comprises a variant Fc region, wherein the variant Fc region consists of or comprises two Fc domains which form a heterodimer, wherein the first Fc domain consists of or comprises the amino acid sequence set forth in SEQ ID NO: 17, and the second Fc domain consists of or comprises the amino acid sequence set forth in SEQ ID NO: 11. In an embodiment, the FcRn antagonist consists of or comprises a variant Fc region, wherein the variant Fc region consists of or comprises two Fc domains which form a heterodimer, wherein the first Fc domain consists of or comprises the amino acid sequence set forth in SEQ ID NO: 17, and the second Fc domain consists of or comprises the amino acid sequence set forth in SEQ ID NO: 14. In an embodiment, the FcRn antagonist consists of or comprises a variant Fc region, wherein the variant Fc region consists of or comprises two Fc domains which form a heterodimer, wherein the first Fc domain consists of or comprises the amino acid sequence set forth in SEQ ID NO: 17, and the second Fc domain consists of or comprises the amino acid sequence set forth in SEQ ID NO: 20. In an embodiment, the FcRn antagonist consists of or comprises a variant Fc region, wherein the variant Fc region consists of or comprises two Fc domains which form a heterodimer, wherein the first Fc domain consists of or comprises the amino acid sequence set forth in SEQ ID NO: 17, and the second Fc domain consists of or comprises the amino acid sequence set forth in SEQ ID NO: 23.

In an embodiment, the FcRn antagonist consists of or comprises a variant Fc region, wherein the variant Fc region consists of or comprises two Fc domains which form a heterodimer, wherein the first Fc domain consists of or comprises an amino acid sequence selected from an amino acid sequence set forth in SEQ ID NOs: 10-12, and the second Fc domain consists of or comprises an amino acid sequence selected from an amino acid sequence set forth in SEQ ID NOs: 7-9. In an embodiment, the FcRn antagonist consists of or comprises a variant Fc region, wherein the variant Fc region consists of or comprises two Fc domains which form a heterodimer, wherein the first Fc domain consists of or comprises an amino acid sequence selected from an amino acid sequence set forth in SEQ ID NOs: 10-12, and the second Fc domain consists of or comprises an amino acid sequence selected from an amino acid sequence set forth in SEQ ID NOs: 13-15. In an embodiment, the FcRn antagonist consists of or comprises a variant Fc region, wherein the variant Fc region consists of or comprises two Fc domains which form a heterodimer, wherein the first Fc domain consists of or comprises an amino acid sequence selected from an amino acid sequence set forth in SEQ ID NOs: 10-12, and the second Fc domain consists of or comprises an amino acid sequence selected from an amino acid sequence set forth in SEQ ID NOs: 19-21. In an embodiment, the FcRn antagonist consists of or comprises a variant Fc region, wherein the variant Fc region consists of or comprises two Fc domains which form a heterodimer, wherein the first Fc domain consists of or comprises an amino acid sequence selected from an amino acid sequence set forth in SEQ ID NOs: 10-12, and the second Fc domain consists of or comprises an amino acid sequence selected from an amino acid sequence set forth in SEQ ID NOs: 22-24.

In an embodiment, the FcRn antagonist consists of or comprises a variant Fc region, wherein the variant Fc region consists of or comprises two Fc domains which form a heterodimer, wherein the first Fc domain consists of or comprises the amino acid sequence set forth in SEQ ID NO: 11, and the second Fc domain consists of or comprises the amino acid sequence set forth in SEQ ID NO: 8. In an embodiment, the FcRn antagonist consists of or comprises a variant Fc region, wherein the variant Fc region consists of or comprises two Fc domains which form a heterodimer, wherein the first Fc domain consists of or comprises the amino acid sequence set forth in SEQ ID NO: 11, and the second Fc domain consists of or comprises the amino acid sequence set forth in SEQ ID NO: 14. In an embodiment, the FcRn antagonist consists of or comprises a variant Fc region, wherein the variant Fc region consists of or comprises two Fc domains which form a heterodimer, wherein the first Fc domain consists of or comprises the amino acid sequence set forth in SEQ ID NO: 11, and the second Fc domain consists of or comprises the amino acid sequence set forth in SEQ ID NO: 20. In an embodiment, the FcRn antagonist consists of or comprises a variant Fc region, wherein the variant Fc region consists of or comprises two Fc domains which form a heterodimer, wherein the first Fc domain consists of or comprises the amino acid sequence set forth in SEQ ID NO: 11, and the second Fc domain consists of or comprises the amino acid sequence set forth in SEQ ID NO: 23.

In an embodiment, the FcRn antagonist comprises a variant Fc region, wherein the variant Fc region consists of or comprises two Fc domains which form a homodimer, wherein the amino acid sequence of each of the Fc domains consists of or comprises the amino acid sequence of SEQ ID NO: 7.

In an embodiment, the FcRn antagonist comprises a variant Fc region, wherein the variant Fc region consists of or comprises two Fc domains which form a homodimer, wherein the amino acid sequence of each of the Fc domains consists of or comprises the amino acid sequence of SEQ ID NO: 8.

In an embodiment, the FcRn antagonist comprises a variant Fc region, wherein the variant Fc region consists of or comprises two Fc domains which form a homodimer, wherein the amino acid sequence of each of the Fc domains consists of or comprises the amino acid sequence of SEQ ID NO: 9.

In an embodiment, the FcRn antagonist comprises a variant Fc region, wherein the variant Fc region consists of or comprises two Fc domains which form a homodimer, wherein the amino acid sequence of each of the Fc domains consists of or comprises the amino acid sequence of SEQ ID NO: 13.

In an embodiment, the FcRn antagonist comprises a variant Fc region, wherein the variant Fc region consists of or comprises two Fc domains which form a homodimer, wherein the amino acid sequence of each of the Fc domains consists of or comprises the amino acid sequence of SEQ ID NO: 14.

In an embodiment, the FcRn antagonist comprises a variant Fc region, wherein the variant Fc region consists of or comprises two Fc domains which form a homodimer, wherein the amino acid sequence of each of the Fc domains consists of or comprises the amino acid sequence of SEQ ID NO: 15.

In an embodiment, the FcRn antagonist comprises a variant Fc region, wherein the variant Fc region consists of or comprises two Fc domains which form a homodimer, wherein the amino acid sequence of each of the Fc domains consists of or comprises the amino acid sequence of SEQ ID NO: 19.

In an embodiment, the FcRn antagonist comprises a variant Fc region, wherein the variant Fc region consists of or comprises two Fc domains which form a homodimer, wherein the amino acid sequence of each of the Fc domains consists of or comprises the amino acid sequence of SEQ ID NO: 20.

In an embodiment, the FcRn antagonist comprises a variant Fc region, wherein the variant Fc region consists of or comprises two Fc domains which form a homodimer, wherein the amino acid sequence of each of the Fc domains consists of or comprises the amino acid sequence of SEQ ID NO: 21.

In an embodiment, the FcRn antagonist comprises a variant Fc region, wherein the variant Fc region consists of or comprises two Fc domains which form a homodimer, wherein the amino acid sequence of each of the Fc domains consists of or comprises the amino acid sequence of SEQ ID NO: 22.

In an embodiment, the FcRn antagonist comprises a variant Fc region, wherein the variant Fc region consists of or comprises two Fc domains which form a homodimer, wherein the amino acid sequence of each of the Fc domains consists of or comprises the amino acid sequence of SEQ ID NO: 23.

In an embodiment, the FcRn antagonist comprises a variant Fc region, wherein the variant Fc region consists of or comprises two Fc domains which form a homodimer, wherein the amino acid sequence of each of the Fc domains consists of or comprises the amino acid sequence of SEQ ID NO: 24.

**Table 1. Amino acid sequences of variant Fc regions**

| **SEQ ID NO:** | **Amino Acid Sequence** |
|---|---|
| 1 | CPPCPAPELLGGPSVFLFPPKPKDTLYITREPEVTCVVVDVSHEDPE VKFNWYVDGVEVHNAKTKPREEQYNSTYRVVSVLTVLHQDWLN GKEYKCKVSNKALPAPIEKTISKAKGQPREPQVYTLPPSRDELTKN |
| | QVSLTCLVKGFYPSDIAVEWESNGQPENNYKTTPPVLDSDGSFFLY SKLTVDKSRWQQGNVFSCSVMHEALKFHYTQKSLSLSPG |
| 2 | DKTHTCPPCPAPELLGGPSVFLFPPKPKDTLYITREPEVTCVVVDVS HEDPEVKFNWYVDGVEVHNAKTKPREEQYNSTYRVVSVLTVLHQ DWLNGKEYKCKVSNKALPAPIEKTISKAKGQPREPQVYTLPPSRDE LTKNQVSLTCLVKGFYPSDIAVEWESNGQPENNYKTTPPVLDSDGS FFLYSKLTVDKSRWQQGNVFSCSVMHEALKFHYTQKSLSLSPGK |
| 3 | DKTHTCPPCPAPELLGGPSVFLFPPKPKDTLYITREPEVTCVVVDVS HEDPEVKFNWYVDGVEVHNAKTKPREEQYNSTYRVVSVLTVLHQ DWLNGKEYKCKVSNKALPAPIEKTISKAKGQPREPQVYTLPPSRDE LTKNQVSLTCLVKGFYPSDIAVEWESNGQPENNYKTTPPVLDSDGS FFLYSKLTVDKSRWQQGNVFSCSVMHEALKFHYTQKSLSLSPG |
| 4 | CPPCPAPELLAGPSVFLFPPKPKDTLMISRTPEVTCVVVDVSHEDPE VKFNWYVDGVEVHNAKTKPREEQYNSTYRVVSVLTVLHQDWLN GKEYKCKVSNKALPAPIEKTISKAKGQPREPQVYTLPPSRDELTKN QVSLTCLVKGFYPSDIAVEWESNGQPENNYKTTPPVLDSDGSFFLY SKLTVDKSRWQQGNVFSCSVMHEALHNHYTQKSLSLSPG |
| 5 | DKTHTCPPCPAPELLAGPSVFLFPPKPKDTLMISRTPEVTCVVVDVS HEDPEVKFNWYVDGVEVHNAKTKPREEQYNSTYRVVSVLTVLHQ DWLNGKEYKCKVSNKALPAPIEKTISKAKGQPREPQVYTLPPSRDE LTKNQVSLTCLVKGFYPSDIAVEWESNGQPENNYKTTPPVLDSDGS FFLYSKLTVDKSRWQQGNVFSCSVMHEALHNHYTQKSLSLSPGK |
| 6 | DKTHTCPPCPAPELLAGPSVFLFPPKPKDTLMISRTPEVTCVVVDVS HEDPEVKFNWYVDGVEVHNAKTKPREEQYNSTYRVVSVLTVLHQ DWLNGKEYKCKVSNKALPAPIEKTISKAKGQPREPQVYTLPPSRDE LTKNQVSLTCLVKGFYPSDIAVEWESNGQPENNYKTTPPVLDSDGS FFLYSKLTVDKSRWQQGNVFSCSVMHEALHNHYTQKSLSLSPG |
| 7 | CPPCPAPELLAGPSVFLFPPKPKDTLYITREPEVTCVVVDVSHEDPE VKFNWYVDGVEVHNAKTKPREEQYNSTYRVVSVLTVLHQDWLN GKEYKCKVSNKALPAPIEKTISKAKGQPREPQVYTLPPSRDELTKN QVSLTCLVKGFYPSDIAVEWESNGQPENNYKTTPPVLDSDGSFFLY SKLTVDKSRWQQGNVFSCSVMHEALKFHYTQKSLSLSPG |
| 8 | DKTHTCPPCPAPELLAGPSVFLFPPKPKDTLYITREPEVTCVVVDVS HEDPEVKFNWYVDGVEVHNAKTKPREEQYNSTYRVVSVLTVLHQ DWLNGKEYKCKVSNKALPAPIEKTISKAKGQPREPQVYTLPPSRDE LTKNQVSLTCLVKGFYPSDIAVEWESNGQPENNYKTTPPVLDSDGS FFLYSKLTVDKSRWQQGNVFSCSVMHEALKFHYTQKSLSLSPGK |
| 9 | DKTHTCPPCPAPELLAGPSVFLFPPKPKDTLYITREPEVTCVVVDVS HEDPEVKFNWYVDGVEVHNAKTKPREEQYNSTYRVVSVLTVLHQ DWLNGKEYKCKVSNKALPAPIEKTISKAKGQPREPQVYTLPPSRDE LTKNQVSLTCLVKGFYPSDIAVEWESNGQPENNYKTTPPVLDSDGS FFLYSKLTVDKSRWQQGNVFSCSVMHEALKFHYTQKSLSLSPG |
| 10 | CPPCPAPDLLGDDSVFLFPPKPKDTLMISRTPEVTCVVVDVSDEDGE VKFNWYVDGVEVHNAKTKPREEQYNSTYRVVSVLTVLHQDWLN GKEYKCKVSNKALPRPIEKTISKAKGQPREPQVYTLPPSRDELTKN QVSLTCLVKGFYPSDIAVEWESNGQPENNYKTTPPVLDSDGSFFLY SKLTVDKSRWQQGNVFSCSVMHEALHNHYTQKSLSLSPG |
| 11 | DKTHTCPPCPAPDLLGDDSVFLFPPKPKDTLMISRTPEVTCVVVDVS DEDGEVKFNWYVDGVEVHNAKTKPREEQYNSTYRVVSVLTVLHQ DWLNGKEYKCKVSNKALPRPIEKTISKAKGQPREPQVYTLPPSRDE LTKNQVSLTCLVKGFYPSDIAVEWESNGQPENNYKTTPPVLDSDGS FFLYSKLTVDKSRWQQGNVFSCSVMHEALHNHYTQKSLSLSPGK |
| 12 | DKTHTCPPCPAPDLLGDDSVFLFPPKPKDTLMISRTPEVTCVVVDVS DEDGEVKFNWYVDGVEVHNAKTKPREEQYNSTYRVVSVLTVLHQ DWLNGKEYKCKVSNKALPRPIEKTISKAKGQPREPQVYTLPPSRDE LTKNQVSLTCLVKGFYPSDIAVEWESNGQPENNYKTTPPVLDSDGS FFLYSKLTVDKSRWQQGNVFSCSVMHEALHNHYTQKSLSLSPG |
| 13 | CPPCPAPDLLGDDSVFLFPPKPKDTLYITREPEVTCVVVDVSDEDGE VKFNWYVDGVEVHNAKTKPREEQYNSTYRVVSVLTVLHQDWLN GKEYKCKVSNKALPRPIEKTISKAKGQPREPQVYTLPPSRDELTKN QVSLTCLVKGFYPSDIAVEWESNGQPENNYKTTPPVLDSDGSFFLY SKLTVDKSRWQQGNVFSCSVMHEALKFHYTQKSLSLSPG |
| 14 | DKTHTCPPCPAPDLLGDDSVFLFPPKPKDTLYITREPEVTCVVVDVS DEDGEVKFNWYVDGVEVHNAKTKPREEQYNSTYRVVSVLTVLHQ DWLNGKEYKCKVSNKALPRPIEKTISKAKGQPREPQVYTLPPSRDE LTKNQVSLTCLVKGFYPSDIAVEWESNGQPENNYKTTPPVLDSDGS FFLYSKLTVDKSRWQQGNVFSCSVMHEALKFHYTQKSLSLSPGK |
| 15 | DKTHTCPPCPAPDLLGDDSVFLFPPKPKDTLYITREPEVTCVVVDVS DEDGEVKFNWYVDGVEVHNAKTKPREEQYNSTYRVVSVLTVLHQ DWLNGKEYKCKVSNKALPRPIEKTISKAKGQPREPQVYTLPPSRDE LTKNQVSLTCLVKGFYPSDIAVEWESNGQPENNYKTTPPVLDSDGS FFLYSKLTVDKSRWQQGNVFSCSVMHEALKFHYTQKSLSLSPG |
| 16 | CPPCPAPELLGGPSVFLFPPKPKDTLMISRTPEVTCVVVDVSHEDPE VKFNWYVDGVEVHNAKTKPREEQYNSTYRVVSVLTVLHQDWLN GKEYKCKVSNKALPAPIEKTISKAKGQPREPQVYTLPPSRDELTKN QVSLTCLVKGFYPSDIAVEWESNGQPENNYKTTPPVLDSDGSFFLY SKLTVDKSRWQQGNVFSCSVMHEALKFHYTQKSLSLSPG |
| 17 | DKTHTCPPCPAPELLGGPSVFLFPPKPKDTLMISRTPEVTCVVVDVS HEDPEVKFNWYVDGVEVHNAKTKPREEQYNSTYRVVSVLTVLHQ DWLNGKEYKCKVSNKALPAPIEKTISKAKGQPREPQVYTLPPSRDE LTKNQVSLTCLVKGFYPSDIAVEWESNGQPENNYKTTPPVLDSDGS FFLYSKLTVDKSRWQQGNVFSCSVMHEALKFHYTQKSLSLSPGK |
| 18 | DKTHTCPPCPAPELLGGPSVFLFPPKPKDTLMISRTPEVTCVVVDVS HEDPEVKFNWYVDGVEVHNAKTKPREEQYNSTYRVVSVLTVLHQ DWLNGKEYKCKVSNKALPAPIEKTISKAKGQPREPQVYTLPPSRDE LTKNQVSLTCLVKGFYPSDIAVEWESNGQPENNYKTTPPVLDSDGS FFLYSKLTVDKSRWQQGNVFSCSVMHEALKFHYTQKSLSLSPG |
| 19 | CPPCPAPELLAGPSVFLFPPKPKDTLMISRTPEVTCVVVDVSHEDPE VKFNWYVDGVEVHNAKTKPREEQYNSTYRVVSVLTVLHQDWLN GKEYKCKVSNKALPAPIEKTISKAKGQPREPQVYTLPPSRDELTKN QVSLTCLVKGFYPSDIAVEWESNGQPENNYKTTPPVLDSDGSFFLY SKLTVDKSRWQQGNVFSCSVMHEALKFHYTQKSLSLSPG |
| 20 | DKTHTCPPCPAPELLAGPSVFLFPPKPKDTLMISRTPEVTCVVVDVS HEDPEVKFNWYVDGVEVHNAKTKPREEQYNSTYRVVSVLTVLHQ |
| | DWLNGKEYKCKVSNKALPAPIEKTISKAKGQPREPQVYTLPPSRDE LTKNQVSLTCLVKGFYPSDIAVEWESNGQPENNYKTTPPVLDSDGS FFLYSKLTVDKSRWQQGNVFSCSVMHEALKFHYTQKSLSLSPGK |
| 21 | DKTHTCPPCPAPELLAGPSVFLFPPKPKDTLMISRTPEVTCVVVDVS HEDPEVKFNWYVDGVEVHNAKTKPREEQYNSTYRVVSVLTVLHQ DWLNGKEYKCKVSNKALPAPIEKTISKAKGQPREPQVYTLPPSRDE LTKNQVSLTCLVKGFYPSDIAVEWESNGQPENNYKTTPPVLDSDGS FFLYSKLTVDKSRWQQGNVFSCSVMHEALKFHYTQKSLSLSPG |
| 22 | CPPCPAPDLLGDDSVFLFPPKPKDTLMISRTPEVTCVVVDVSDEDGE VKFNWYVDGVEVHNAKTKPREEQYNSTYRVVSVLTVLHQDWLN GKEYKCKVSNKALPRPIEKTISKAKGQPREPQVYTLPPSRDELTKN QVSLTCLVKGFYPSDIAVEWESNGQPENNYKTTPPVLDSDGSFFLY SKLTVDKSRWQQGNVFSCSVMHEALKFHYTQKSLSLSPG |
| 23 | DKTHTCPPCPAPDLLGDDSVFLFPPKPKDTLMISRTPEVTCVVVDVS DEDGEVKFNWYVDGVEVHNAKTKPREEQYNSTYRVVSVLTVLHQ DWLNGKEYKCKVSNKALPRPIEKTISKAKGQPREPQVYTLPPSRDE LTKNQVSLTCLVKGFYPSDIAVEWESNGQPENNYKTTPPVLDSDGS FFLYSKLTVDKSRWQQGNVFSCSVMHEALKFHYTQKSLSLSPGK |
| 24 | DKTHTCPPCPAPDLLGDDSVFLFPPKPKDTLMISRTPEVTCVVVDVS DEDGEVKFNWYVDGVEVHNAKTKPREEQYNSTYRVVSVLTVLHQ DWLNGKEYKCKVSNKALPRPIEKTISKAKGQPREPQVYTLPPSRDE LTKNQVSLTCLVKGFYPSDIAVEWESNGQPENNYKTTPPVLDSDGS FFLYSKLTVDKSRWQQGNVFSCSVMHEALKFHYTQKSLSLSPG |

In an aspect, the FcRn antagonist is an anti-FcRn antibody comprising amino acid A at EU position 236. In another aspect, the FcRn antagonist is an anti-FcRn antibody comprising amino acids D, D, D, D, G, and R, at EU positions 233, 237, 238, 268, 271, and 330, respectively. In another aspect, the FcRn antagonist is an anti-FcRn antibody comprising amino acid A at EU position 236 and amino acids D, D, D, D, G, and R, at EU positions 233, 237, 238, 268, 271, and 330, respectively. In some embodiments, the anti-FcRn antibody is afucosylated.

### Polypeptides

In another aspect, provided herein are polypeptides comprising an Fc domain comprising amino acids A, Y, T, E, K, F, and Y at EU positions 236, 252, 254, 256, 433, 434, and 436, respectively. In another aspect, provided herein are polypeptides comprising an Fc domain comprising amino acids A, K, F, and Y at EU positions 236, 433, 434, and 436, respectively. In another aspect, provided herein are polypeptides comprising an Fc domain comprising amino acids A, Y, T, E, K, F, and F at EU positions 236, 252, 254, 256, 433, 434, and 436, respectively. In another aspect, provided herein are polypeptides comprising an Fc domain comprising amino acids A, K, F, and F at EU positions 236, 433, 434, and 436, respectively. In some embodiments, the polypeptide is afucosylated.

In another aspect, provided herein are polypeptides comprising an Fc domain comprising amino acids D, D, D, Y, T, E, D, G, R, K, F, and Y at EU positions 233, 237, 238, 252, 254, 256, 268, 271, 330, 433, 434, and 436, respectively. In another aspect, provided herein are polypeptides comprising an Fc domain comprising amino acids D, D, D, D, G, R, K, F, and Y at EU positions 233, 237, 238, 268, 271, 330, 433, 434, and 436, respectively. In another aspect, provided herein are polypeptides comprising an Fc domain comprising amino acids D, D, D, Y, T, E, D, G, R, K, F, and F at EU positions 233, 237, 238, 252, 254, 256, 268, 271, 330, 433, 434, and 436, respectively. In another aspect, provided herein are polypeptides comprising an Fc domain comprising amino acids D, D, D, D, G, R, K, F, and F at EU positions 233, 237, 238, 268, 271, 330, 433, 434, and 436, respectively. In some embodiments, the polypeptide is afucosylated.

In another aspect, provided herein are polypeptides comprising an Fc domain comprising amino acids D, A, D, D, Y, T, E, D, G, R, K, F, and Y at EU positions 233, 236, 237, 238, 252, 254, 256, 268, 271, 330, 433, 434, and 436, respectively. In another aspect, provided herein are polypeptides comprising an Fc domain comprising amino acids D, A, D, D, D, G, R, K, F, and Y at EU positions 233, 236, 237, 238, 268, 271, 330, 433, 434, and 436, respectively. In another aspect, provided herein are polypeptides comprising an Fc domain comprising amino acids D, A, D, D, Y, T, E, D, G, R, K, F, and F at EU positions 233, 236, 237, 238, 252, 254, 256, 268, 271, 330, 433, 434, and 436, respectively. In another aspect, provided herein are polypeptides comprising an Fc domain comprising amino acids D, A, D, D, D, G, R, K, F, and F at EU positions 233, 236, 237, 238, 268, 271, 330, 433, 434, and 436, respectively. In some embodiments, the polypeptide is afucosylated.

In another aspect, provided herein are polypeptides comprising one or more sequences set forth in Table 1. In some embodiments, the polypeptide comprises or consists of an amino acid sequence set forth in SEQ ID NO: 7. In some embodiments, the polypeptide comprises or consists of an amino acid sequence set forth in SEQ ID NO: 8. In some embodiments, the polypeptide comprises or consists of an amino acid sequence set forth in SEQ ID NO: 9. In some embodiments, the polypeptide comprises or consists of an amino acid sequence set forth in SEQ ID NO: 13. In some embodiments, the polypeptide comprises or consists of an amino acid sequence set forth in SEQ ID NO: 14. In some embodiments, the polypeptide comprises or consists of an amino acid sequence set forth in SEQ ID NO: 15. In some embodiments, the polypeptide comprises or consists of an amino acid sequence set forth in SEQ ID NO: 19. In some embodiments, the polypeptide comprises or consists of an amino acid sequence set forth in SEQ ID NO: 20. In some embodiments, the polypeptide comprises or consists of an amino acid sequence set forth in SEQ ID NO: 21. In some embodiments, the polypeptide comprises or consists of an amino acid sequence set forth in SEQ ID NO: 22. In some embodiments, the polypeptide comprises or consists of an amino acid sequence set forth in SEQ ID NO: 23. In some embodiments, the polypeptide comprises or consists of an amino acid sequence set forth in SEQ ID NO: 24. In some embodiments, the polypeptide is afucosylated.

### Polynucleotides, Vectors, and Methods of Production

The disclosure also provides polynucleotides encoding the FcRn antagonists disclosed herein or fragments thereof. In some embodiments, a polynucleotide described herein is isolated or purified. As used herein, an "isolated" polynucleotide or nucleic acid molecule is one which is separated from other nucleic acid molecules which are present in the natural source (*e.g*., in a mouse or a human) of the nucleic acid molecule. Moreover, an "isolated" nucleic acid molecule, such as a cDNA molecule, can be substantially free of other cellular material, or culture medium when produced by recombinant techniques, or substantially free of chemical precursors or other chemicals when chemically synthesized. For example, the language "substantially free" includes preparations of polynucleotide or nucleic acid molecules having less than about 15%, 10%, 5%, 2%, 1%, 0.5%, or 0.1% (in particular, less than about 10%) of other material, *e.g*., cellular material, culture medium, other nucleic acid molecules, chemical precursors and/or other chemicals. In an embodiment, a nucleic acid molecule(s) encoding a polypeptide described herein is isolated or purified.

In an aspect, provided herein are polynucleotides comprising a nucleotide sequence encoding an FcRn antagonist described herein. In some embodiments, the polynucleotides comprise a nucleotide sequence that encodes an Fc domain described herein. In some embodiments, the polynucleotides comprise a nucleotide sequence that encodes an Fc domain comprising the amino acid sequence of any one of SEQ ID NOs: 1-24. In some embodiments, the polynucleotides comprise a nucleotide sequence that encodes an Fc domain consisting of the amino acid sequence of any one of SEQ ID NOs: 1-24.

In some embodiments, the polynucleotides comprise nucleotide sequences that encode two or more Fc domains. In some embodiments, the polynucleotides comprise nucleotide sequences that encode two Fc domains. In some embodiments, the polynucleotides comprise a first nucleotide sequence that encodes a first Fc domain and a second nucleotide sequence that encodes a second Fc domain. In some embodiments, the first nucleotide sequence and the second nucleotide sequence are comprised in distinct nucleic acid molecules. In some embodiments, the first nucleotide sequence and the second nucleotide sequence are comprised in the same nucleic acid molecule.

In some embodiments, the first and second nucleotide sequence encode different Fc domains. In some embodiments, the first nucleotide sequence encodes a first Fc domain described herein and the second nucleotide sequence encodes a second Fc domain described herein. In some embodiments, the first nucleotide sequence encodes an Fc domain comprising or consisting of the amino acid sequence of any one of SEQ ID NOs: 1-3 and the second nucleotide sequence encodes an Fc domain comprising or consisting of the amino acid sequence of any one of SEQ ID NOs: 4-15 or 19-24.

In some embodiments, the first nucleotide sequence encodes an Fc domain comprising or consisting of the amino acid sequence of any one of SEQ ID NOs: 4-6 and the second nucleotide sequence encodes an Fc domain comprising or consisting of the amino acid sequence of any one of SEQ ID NOs: 7-9. In some embodiments, the first nucleotide sequence encodes an Fc domain comprising or consisting of the amino acid sequence of any one of SEQ ID NOs: 4-6 and the second nucleotide sequence encodes an Fc domain comprising or consisting of the amino acid sequence of any one of SEQ ID NOs: 13-15. In some embodiments, the first nucleotide sequence encodes an Fc domain comprising or consisting of the amino acid sequence of any one of SEQ ID NOs: 4-6 and the second nucleotide sequence encodes an Fc domain comprising or consisting of the amino acid sequence of any one of SEQ ID NOs: 19-21. In some embodiments, the first nucleotide sequence encodes an Fc domain comprising or consisting of the amino acid sequence of any one of SEQ ID NOs: 4-6 and the second nucleotide sequence encodes an Fc domain comprising or consisting of the amino acid sequence of any one of SEQ ID NOs: 22-24.

In some embodiments, the first nucleotide sequence encodes an Fc domain comprising or consisting of the amino acid sequence of any one of SEQ ID NOs: 16-18 and the second nucleotide sequence encodes an Fc domain comprising or consisting of the amino acid sequence of any one of SEQ ID NOs: 4-15 or 19-24.

In some embodiments, the first nucleotide sequence encodes an Fc domain comprising or consisting of the amino acid sequence of any one of SEQ ID NOs: 10-12 and the second nucleotide sequence encodes an Fc domain comprising or consisting of the amino acid sequence of any one of SEQ ID NOs: 7-9. In some embodiments, the first nucleotide sequence encodes an Fc domain comprising or consisting of the amino acid sequence of any one of SEQ ID NOs: 10-12 and the second nucleotide sequence encodes an Fc domain comprising or consisting of the amino acid sequence of any one of SEQ ID NOs: 13-15. In some embodiments, the first nucleotide sequence encodes an Fc domain comprising or consisting of the amino acid sequence of any one of SEQ ID NOs: 10-12 and the second nucleotide sequence encodes an Fc domain comprising or consisting of the amino acid sequence of any one of SEQ ID NOs: 19-21. In some embodiments, the first nucleotide sequence encodes an Fc domain comprising or consisting of the amino acid sequence of any one of SEQ ID NOs: 10-12 and the second nucleotide sequence encodes an Fc domain comprising or consisting of the amino acid sequence of any one of SEQ ID NOs: 22-24.

In some embodiments, both the first and second nucleotide sequence encode an Fc domain comprising or consisting of the amino acid sequence of any one of SEQ ID NOs: 7-9. In some embodiments, both the first and second nucleotide sequence encode an Fc domain comprising or consisting of the amino acid sequence of any one of SEQ ID NOs: 13-15. In some embodiments, both the first and second nucleotide sequence encode an Fc domain comprising or consisting of the amino acid sequence of any one of SEQ ID NOs: 19-21. In some embodiments, both the first and second nucleotide sequence encode an Fc domain comprising or consisting of the amino acid sequence of any one of SEQ ID NOs: 22-24.

Also provided herein are polynucleotides encoding a polypeptide as provided above that are optimized, *e.g.*, by codon/RNA optimization, replacement with heterologous signal sequences, and elimination of mRNA instability elements. Methods to generate optimized nucleic acids for recombinant expression by introducing codon changes and/or eliminating inhibitory regions in the mRNA can be carried out by adapting the optimization methods described in, *e.g.*, U.S. Patent Nos. 5,965,726; 6,174,666; 6,291,664; 6,414,132; and 6,794,498, accordingly, all of which are herein incorporated by reference in their entireties. For example, potential splice sites and instability elements (*e.g*., A/T or A/U rich elements) within the RNA can be mutated without altering the amino acids encoded by the nucleic acid sequences to increase stability of the RNA for recombinant expression. The alterations utilize the degeneracy of the genetic code, *e.g.*, using an alternative codon for an identical amino acid. In an embodiment, it can be desirable to alter one or more codons to encode a conservative mutation, *e.g.*, a similar amino acid with similar chemical structure and properties and/or function as the original amino acid.

The polynucleotides can be obtained, and the nucleotide sequence of the polynucleotides determined, by any method known in the art. Nucleotide sequences encoding proteins described herein, and modified versions of these antibodies can be determined using methods well known in the art, *i.e.,* nucleotide codons known to encode particular amino acids are assembled in such a way to generate a nucleic acid that encodes the protein. Such a polynucleotide encoding the protein can be assembled from chemically synthesized oligonucleotides (*e.g*., as described in Kutmeier G et al., (1994) BioTechniques 17: 242-6, herein incorporated by reference in its entirety), which, briefly, involves the synthesis of overlapping oligonucleotides containing portions of the sequence encoding the antibody, annealing, and ligating of those oligonucleotides, and then amplification of the ligated oligonucleotides by PCR.

Alternatively, a polynucleotide encoding a protein described herein can be generated from nucleic acid from a suitable source (*e.g.,* a hybridoma) using methods well known in the art (*e.g.,* PCR and other molecular cloning methods). For example, PCR amplification using synthetic primers hybridizable to the 3' and 5' ends of a known sequence can be performed using genomic DNA obtained from hybridoma cells producing the polypeptide of interest. Such PCR amplification methods can be used to obtain nucleic acids comprising the sequence encoding the polypeptide. The amplified nucleic acids can be cloned into vectors for expression in host cells and for further cloning.

If a clone containing a nucleic acid encoding a particular polypeptide is not available, but the sequence of the polypeptide is known, a nucleic acid encoding the polypeptide can be chemically synthesized or obtained from a suitable source (*e.g.,* a cDNA library generated from, or nucleic acid, preferably poly A+ RNA, isolated from any tissue or cells expressing the polypeptide described herein) by PCR amplification using synthetic primers hybridizable to the 3' and 5' ends of the sequence or by cloning using an oligonucleotide probe specific for the particular gene sequence to identify, *e.g.*, a cDNA clone from a cDNA library that encodes the polypeptide. Amplified nucleic acids generated by PCR can then be cloned into replicable cloning vectors using any method well known in the art.

DNA encoding proteins described herein can be readily isolated and sequenced using conventional procedures. Hybridoma cells can serve as a source of such DNA. Once isolated, the DNA can be placed into expression vectors, which are then transfected into host cells such as *E. coli* cells, simian COS cells, Chinese hamster ovary (CHO) cells (*e.g.,* CHO cells from the CHO GS System^{™} (Lonza)), or myeloma cells that do not otherwise produce the proteins described herein.

Also provided are polynucleotides that hybridize under high stringency, intermediate or lower stringency hybridization conditions to polynucleotides that encode a protein described herein.

Hybridization conditions have been described in the art and are known to one of skill in the art. For example, hybridization under stringent conditions can involve hybridization to filter-bound DNA in 6x sodium chloride/sodium citrate (SSC) at about 45° C followed by one or more washes in 0.2xSSC/0.1% SDS at about 50-65° C; hybridization under highly stringent conditions can involve hybridization to filter-bound nucleic acid in 6xSSC at about 45° C followed by one or more washes in 0.1xSSC/0.2% SDS at about 68° C. Hybridization under other stringent hybridization conditions is known to those of skill in the art and has been described, *see, e.g.*, Ausubel FM et al., eds., (1989) Current Protocols in Molecular Biology, Vol. I, Green Publishing Associates, Inc. and John Wiley & Sons, Inc., New York at pages 6.3.1-6.3.6 and 2.10.3, which is herein incorporated by reference in its entirety.

In an aspect, provided herein are cells (*e.g.,* host cells) expressing (*e.g.,* recombinantly) a protein described herein, and related polynucleotides and expression vectors. Provided herein are vectors (*e.g*., expression vectors) comprising polynucleotides comprising nucleotide sequences encoding a protein described herein for recombinant expression in host cells, preferably in mammalian cells (*e.g.,* CHO cells). Also provided herein are host cells comprising such vectors for recombinantly expressing proteins described herein. In an aspect, provided herein are methods for producing a protein described herein, comprising expressing the polypeptide from a host cell.

Recombinant expression of a protein described herein generally involves construction of an expression vector containing a polynucleotide that encodes the polypeptide. Once a polynucleotide encoding a polypeptide described herein has been obtained, the vector for the production of the polypeptide can be produced by recombinant DNA technology using techniques well known in the art. Thus, methods for preparing a protein by expressing a polynucleotide containing a polypeptide encoding nucleotide sequence are described herein. Methods which are well known to those skilled in the art can be used to construct expression vectors containing polypeptide coding sequences and appropriate transcriptional and translational control signals. These methods include, for example, *in vitro* recombinant DNA techniques, synthetic techniques, and *in vivo* genetic recombination. Also provided are replicable vectors comprising a nucleotide sequence encoding containing a polypeptide described herein, operably linked to a promoter. Such vectors can, for example, include a nucleotide sequence encoding a first Fc domain of the disclosure (*see, e.g.*, International Publication Nos. WO 86/05807 and WO 89/01036; and U.S. Patent No. 5,122,464, which are herein incorporated by reference in their entireties), and a second Fc domain of the disclosure can be cloned into such a vector for expression of the first Fc domain, the second Fc domain, or both the first and second Fc domains.

An expression vector can be transferred to a cell (*e.g.,* host cell) by conventional techniques and the resulting cells can then be cultured by conventional techniques to produce a polypeptide described herein or a fragment thereof. Thus, provided herein are host cells containing a polynucleotide encoding containing a polypeptide described herein or fragments thereof, or a heavy or light chain thereof, or fragment thereof, operably linked to a promoter for expression of such sequences in the host cell.

In an embodiment, a host cell comprises a polynucleotide comprising one of the first nucleotide sequences and one of the second nucleotide sequences described above. In another embodiment, a host cell comprises a first polynucleotide comprising one of the first nucleotide sequences described above, and a second polynucleotide comprising one of the first nucleotide sequences described above. In another embodiment, a host cell comprises a first vector comprising one of the first nucleotide sequences and one of the second nucleotide sequences described above. In another embodiment, a host cell comprises a first vector comprising one of the first nucleotide sequences and one of the second nucleotide sequences described above, and a second vector comprising a second polynucleotide comprising one of the first nucleotide sequences described above.

In some embodiments, an Fc domain expressed by a first host cell is associated with an Fc domain expressed by a second host cell to form an FcRn antagonist. In some embodiments, provided herein are populations of host cells comprising such first host cells and such second host cells.

In some embodiments, provided herein is a population of vectors comprising a first vector comprising a polynucleotide encoding an Fc domain, and a second vector comprising a polynucleotide encoding an Fc domain. In some embodiments, provided herein is a population of vectors comprising a first vector comprising a polynucleotide encoding an Fc domain and a polynucleotide encoding an Fc domain. In some embodiments, provided herein is a population of vectors comprising a first vector comprising a polynucleotide encoding two Fc domains.

A variety of host-expression vector systems can be utilized to express polypeptides described herein (*see, e.g.*, U.S. Patent No. 5,807,715, which is herein incorporated by reference in its entirety). Such host-expression systems represent vehicles by which the coding sequences of interest can be produced and subsequently purified, but also represent cells which can, when transformed or transfected with the appropriate nucleotide coding sequences, express a polypeptide described herein in situ. These include but are not limited to microorganisms such as bacteria (*e.g., E. coli* and *B. subtilis*) transformed with, *e.g.*, recombinant bacteriophage DNA, plasmid DNA, or cosmid DNA expression vectors containing Fc domain coding sequences; yeast (*e.g*., Saccharomyces and Pichia) transformed with, *e.g.*, recombinant yeast expression vectors containing Fc domain coding sequences; insect cell systems infected with, *e.g.*, recombinant virus expression vectors (*e.g*., baculovirus) containing Fc domain coding sequences; plant cell systems (*e.g.,* green algae such as *Chlamydomonas reinhardtii)* infected with, *e.g.*, recombinant virus expression vectors (*e.g*., cauliflower mosaic virus, CaMV; tobacco mosaic virus, TMV) or transformed with, *e.g.*, recombinant plasmid expression vectors (*e.g.,* Ti plasmid) containing Fc domain coding sequences; or mammalian cell systems (*e.g.,* COS (*e.g.,* COS1 or COS), CHO, BHK, MDCK, HEK 293, NS0, PER.C6, VERO, CRL7O3O, HsS78Bst, HeLa, NIH 3T3, HEK-293T, HepG2, SP210, R1.1, B-W, L-M, BSC1, BSC40, YB/20, and BMT10 cells) harboring, *e.g.*, recombinant expression constructs containing promoters derived from the genome of mammalian cells (*e.g.,* metallothionein promoter) or from mammalian viruses (*e.g.,* the adenovirus late promoter; the vaccinia virus 7.5K promoter). In an embodiment, cells for expressing Fc domains described herein are Chinese hamster ovary (CHO) cells, for example CHO cells from the CHO GS System^{™} (Lonza). In an embodiment, the heavy chain and/or light chain produced by a CHO cell may have an N-terminal glutamine or glutamate residue replaced by pyroglutamate. In an embodiment, cells for expressing polypeptides described herein are human cells, *e.g.*, human cell lines. In an embodiment, a mammalian expression vector is pOptiVEC^{™} or pcDNA3.3. In an embodiment, bacterial cells such as *Escherichia coli,* or eukaryotic cells (*e.g.,* mammalian cells), are used for the expression of a recombinant polypeptide. For example, mammalian cells such as CHO cells, in conjunction with a vector such as the major intermediate early gene promoter element from human cytomegalovirus, are an effective expression system for antibodies (Foecking MK & Hofstetter H (1986) Gene 45: 101-5; and Cockett MI et al., (1990) Biotechnology 8(7): 662-7, each of which is herein incorporated by reference in its entirety). In an embodiment, polypeptides described herein are produced by CHO cells or NS0 cells. In an embodiment, the expression of nucleotide sequences encoding polypeptides described herein which comprise two, three, or four binding sites for human FcRn is regulated by a constitutive promoter, inducible promoter, or tissue specific promoter.

In bacterial systems, a number of expression vectors can be advantageously selected depending upon the use intended for the molecule being expressed. For example, when a large quantity of such a polypeptide is to be produced, for the generation of pharmaceutical compositions of an antibody molecule, vectors which direct the expression of high levels of fusion protein products that are readily purified can be desirable. Such vectors include, but are not limited to, the *E. coli* expression vector pUR278 (Ruether U & Mueller-Hill B (1983) EMBO J 2: 1791-1794), in which the coding sequence can be ligated individually into the vector in frame with the lac Z coding region so that a fusion protein is produced; pIN vectors (Inouye S & Inouye M (1985) Nuc Acids Res 13: 3101-3109; Van Heeke G & Schuster SM (1989) J Biol Chem 24: 5503-5509); and the like, all of which are herein incorporated by reference in their entireties. For example, pGEX vectors can also be used to express foreign polypeptides as fusion proteins with glutathione 5-transferase (GST). In general, such fusion proteins are soluble and can easily be purified from lysed cells by adsorption and binding to matrix glutathione agarose beads followed by elution in the presence of free glutathione. The pGEX vectors are designed to include thrombin or factor Xa protease cleavage sites so that the cloned target gene product can be released from the GST moiety.

In an insect system, *Autographa californica* nuclear polyhedrosis virus (AcNPV), for example, can be used as a vector to express foreign genes. The virus grows in *Spodoptera frugiperda* cells. The coding sequence can be cloned individually into non-essential regions (for example the polyhedrin gene) of the virus and placed under control of an AcNPV promoter (for example the polyhedrin promoter).

In mammalian host cells, a number of viral-based expression systems can be utilized. In cases where an adenovirus is used as an expression vector, the coding sequence of interest can be ligated to an adenovirus transcription/translation control complex, *e.g.*, the late promoter and tripartite leader sequence. This chimeric gene can then be inserted in the adenovirus genome by *in vitro* or *in vivo* recombination. Insertion in a non-essential region of the viral genome (*e.g.,* region E1 or E3) will result in a recombinant virus that is viable and capable of expressing the molecule in infected hosts (*see, e.g.*, Logan J & Shenk T (1984) PNAS 81(12): 3655-9, which is herein incorporated by reference in its entirety). Specific initiation signals can also be required for efficient translation of inserted coding sequences. These signals include the ATG initiation codon and adjacent sequences. Furthermore, the initiation codon must be in phase with the reading frame of the desired coding sequence to ensure translation of the entire insert. These exogenous translational control signals and initiation codons can be of a variety of origins, both natural and synthetic. The efficiency of expression can be enhanced by the inclusion of appropriate transcription enhancer elements, transcription terminators, *etc*. (*see, e.g.*, Bitter G et al., (1987) Methods Enzymol. 153: 516-544, which is herein incorporated by reference in its entirety).

In addition, a host cell strain can be chosen which modulates the expression of the inserted sequences or modifies and processes the gene product in the specific fashion desired. Such modifications (*e.g.,* glycosylation) and processing (*e.g.,* cleavage) of protein products can be important for the function of the protein. Different host cells have characteristic and specific mechanisms for the post-translational processing and modification of proteins and gene products. Appropriate cell lines or host systems can be chosen to ensure the correct modification and processing of the foreign protein expressed. To this end, eukaryotic host cells which possess the cellular machinery for proper processing of the primary transcript, glycosylation, and phosphorylation of the gene product can be used. Such mammalian host cells include but are not limited to CHO, VERO, BHK, HeLa, MDCK, HEK 293, NIH 3T3, W138, BT483, Hs578T, HTB2, BT2O, and T47D, NSO (a murine myeloma cell line that does not endogenously produce any immunoglobulin chains), CRL7O3O, COS (*e.g.,* COS1 or COS), PER.C6, VERO, HsS78Bst, HEK-293T, HepG2, SP210, R1.1, B-W, L-M, BSC1, BSC40, YB/20, BMT10, and HsS78Bst cells. In an embodiment, proteins described herein are produced in mammalian cells, such as CHO cells.

In an embodiment, a polypeptide described herein comprises a portion of an antibody with reduced fucose content or no fucose content (e.g., afucosylated). Such proteins can be produced using techniques known to one skilled in the art. For example, the proteins can be expressed in cells deficient in or lacking the ability to fucosylate. In an example, cell lines with a knockout of both alleles of α1,6-fucosyltransferase can be used to produce antibodies with reduced fucose content. The Potelligent^{®} system (Lonza) is an example of such a system that can be used to produce antibodies with reduced fucose content.

For long-term, high-yield production of recombinant proteins, stable expression cells can be generated. For example, cell lines which stably express a protein described herein can be engineered. In an embodiment, a cell provided herein stably expresses a first Fc domain and a second Fc domain which associate to form an FcRn antagonist described herein.

In certain aspects, rather than using expression vectors which contain viral origins of replication, host cells can be transformed with DNA controlled by appropriate expression control elements (*e.g*., promoter, enhancer, sequences, transcription terminators, polyadenylation sites, *etc.*)*,* and a selectable marker. Following the introduction of the foreign DNA/polynucleotide, engineered cells can be allowed to grow for one to two days in an enriched media, and then are switched to a selective media. The selectable marker in the recombinant plasmid confers resistance to the selection and allows cells to stably integrate the plasmid into their chromosomes and grow to form foci, which in turn can be cloned and expanded into cell lines. This method can advantageously be used to engineer cell lines which express a polypeptide comprising two, three, or four binding sites for human FcRn described herein or a fragment thereof. Such engineered cell lines can be particularly useful in the screening and evaluation of compositions that interact directly or indirectly with the polypeptide.

A number of selection systems can be used, including but not limited to the herpes simplex virus thymidine kinase (Wigler M et al., (1977) Cell 11(1): 223-32), hypoxanthineguanine phosphoribosyltransferase (Szybalska EH & Szybalski W (1962) PNAS 48(12): 2026-2034) and adenine phosphoribosyltransferase (Lowy I et al., (1980) Cell 22(3): 817-23) genes in tk-, hgprt- or aprt-cells, respectively, all of which are herein incorporated by reference in their entireties. Also, antimetabolite resistance can be used as the basis of selection for the following genes: dhfr, which confers resistance to methotrexate (Wigler M et al., (1980) PNAS 77(6): 3567-70; O'Hare K et al., (1981) PNAS 78: 1527-31); gpt, which confers resistance to mycophenolic acid (Mulligan RC & Berg P (1981) PNAS 78(4): 2072-6); neo, which confers resistance to the aminoglycoside G-418 (Wu GY & Wu CH (1991) Biotherapy 3: 87-95; Tolstoshev P (1993) Ann Rev Pharmacol Toxicol 32: 573-596; Mulligan RC (1993) Science 260: 926-932; and Morgan RA & Anderson WF (1993) Ann Rev Biochem 62: 191-217; Nabel GJ & Felgner PL (1993) Trends Biotechnol 11(5): 211-5); and hygro, which confers resistance to hygromycin (Santerre RF et al., (1984) Gene 30(1-3): 147-56), all of which are herein incorporated by reference in their entireties. Methods commonly known in the art of recombinant DNA technology can be routinely applied to select the desired recombinant clone and such methods are described, for example, in Ausubel FM et al., (eds.), Current Protocols in Molecular Biology, John Wiley & Sons, NY (1993); Kriegler M, Gene Transfer and Expression, A Laboratory Manual, Stockton Press, NY (1990); and in Chapters 12 and 13, Dracopoli NC et al., (eds.), Current Protocols in Human Genetics, John Wiley & Sons, NY (1994); Colbère-Garapin F et al., (1981) J Mol Biol 150: 1-14, all of which are herein incorporated by reference in their entireties.

The expression levels of a polypeptide can be increased by vector amplification (for a review, *see,* Bebbington CR & Hentschel CCG, The use of vectors based on gene amplification for the expression of cloned genes in mammalian cells in DNA cloning, p. 163-188. In DNA Cloning, Vol III, A Practical Approach. D. M. Glover (Ed.) (Academic Press, New York, 1987), which is herein incorporated by reference in its entirety). When a marker in the vector system is amplifiable, increase in the level of inhibitor present in culture of host cell will increase the number of copies of the marker gene. Since the amplified region is associated with the gene of interest, production of the polypeptide will also increase (Crouse GF et al., (1983) Mol Cell Biol 3: 257-66, which is herein incorporated by reference in its entirety).

The host cell can be co-transfected with two or more expression vectors described herein. The two vectors can contain identical selectable markers which enable equal expression of polypeptides, such as a first heavy chain or Fc domain and a second heavy chain or Fc domain polypeptide. The host cells can be co-transfected with different amounts of the two or more expression vectors. For example, host cells can be transfected with any one of the following ratios of a first expression vector and a second expression vector: about 1:1, 1:2, 1:3, 1:4, 1:5, 1:6, 1:7, 1:8, 1:9, 1:10, 1:12, 1:15, 1:20, 1:25, 1:30, 1:35, 1:40, 1:45, or 1:50.

Alternatively, a single vector can be used which encodes, and is capable of expressing, both polypeptides. The coding sequences can comprise cDNA or genomic DNA. The expression vector can be monocistronic or multicistronic. A multicistronic nucleic acid construct can encode 2, 3, 4, 5, 6, 7, 8, 9, 10, or more genes/nucleotide sequences, or in the range of 2-5, 5-10, or 10-20 genes/nucleotide sequences. For example, a bicistronic nucleic acid construct can comprise, in the following order, a promoter, a first gene and a second gene. In such an expression vector, the transcription of both genes can be driven by the promoter, whereas the translation of the mRNA from the first gene can be by a cap-dependent scanning mechanism, and the translation of the mRNA from the second gene can be by a cap-independent mechanism, *e.g.*, by an IRES.

Once a polypeptide described herein has been produced by recombinant expression, it can be purified by any method known in the art for purification of a protein, for example, by chromatography (*e.g*., ion exchange, affinity, particularly by affinity for the specific antigen after Protein A, and sizing column chromatography), centrifugation, differential solubility, or by any other standard technique for the purification of proteins. Further, the polypeptides described herein can be fused to heterologous polypeptide sequences described herein or otherwise known in the art to facilitate purification.

In an embodiment, a polypeptide described herein is isolated or purified. In an embodiment, an isolated polypeptide is one that is substantially free of other polypeptides with different antigenic specificities than the isolated polypeptide. For example, in certain embodiments, a preparation of a protein described herein is substantially free of cellular material and/or chemical precursors. The language "substantially free of cellular material" includes preparations of a polypeptide in which the polypeptide is separated from cellular components of the cells from which it is isolated or recombinantly produced. Thus, a polypeptide that is substantially free of cellular material includes preparations of polypeptide having less than about 30%, 20%, 10%, 5%, 2%, 1%, 0.5%, or 0.1% (by dry weight) of heterologous protein (also referred to herein as a "contaminating protein") and/or variants of a polypeptide, for example, different post-translational modified forms of a polypeptide or other different versions of a polypeptide (*e.g*., polypeptide fragments). When the polypeptide is recombinantly produced, it is also generally substantially free of culture medium, *i.e.,* culture medium represents less than about 20%, 10%, 2%, 1 %, 0.5%, or 0.1 % of the volume of the protein preparation. When the polypeptide is produced by chemical synthesis, it is generally substantially free of chemical precursors or other chemicals, *i.e.,* it is separated from chemical precursors or other chemicals, which are involved in the synthesis of the protein. Accordingly, such preparations of the protein have less than about 30%, 20%, 10%, or 5% (by dry weight) of chemical precursors or compounds other than the molecule of interest. In an embodiment, polypeptides described herein are isolated or purified.

A polypeptide described herein can be produced by any method known in the art for the synthesis of proteins, for example, by chemical synthesis or by recombinant expression techniques. The methods described herein employ, unless otherwise indicated, conventional techniques in molecular biology, microbiology, genetic analysis, recombinant DNA, organic chemistry, biochemistry, PCR, oligonucleotide synthesis and modification, nucleic acid hybridization, and related fields within the skill of the art. These techniques are described, for example, in the references cited herein and are fully explained in the literature. *See, e.g.*, Maniatis T et al., (1982) Molecular Cloning: A Laboratory Manual, Cold Spring Harbor Laboratory Press; Sambrook J et al., (1989), Molecular Cloning: A Laboratory Manual, Second Edition, Cold Spring Harbor Laboratory Press; Sambrook J et al., (2001) Molecular Cloning: A Laboratory Manual, Cold Spring Harbor Laboratory Press, Cold Spring Harbor, NY; Ausubel FM et al., Current Protocols in Molecular Biology, John Wiley & Sons (1987 and annual updates); Current Protocols in Immunology, John Wiley & Sons (1987 and annual updates); Gait (ed.) (1984) Oligonucleotide Synthesis: A Practical Approach, IRL Press; Eckstein (ed.) (1991) Oligonucleotides and Analogues: A Practical Approach, IRL Press; Birren B et al., (eds.) (1999) Genome Analysis: A Laboratory Manual, Cold Spring Harbor Laboratory Press, all of which are herein incorporated by reference in their entireties.

In an embodiment, a polypeptide described herein is prepared, expressed, created, or isolated by any means that involves creation, *e.g.*, via synthesis, genetic engineering of DNA sequences. In an embodiment, such a polypeptide comprises sequences (*e.g*., DNA sequences or amino acid sequences) that do not naturally exist within the antibody germline repertoire of an animal or mammal (*e.g.,* human) *in vivo.*

### Pharmaceutical Compositions

In an aspect, the instant disclosure provides pharmaceutical compositions comprising an FcRn antagonist as disclosed herein for use in methods of treating an antibody-mediated disorder (*e.g*., an autoantibody-mediated disorder). In general, these FcRn antagonists inhibit the binding of Fc-containing agents (*e.g.,* antibodies and immunoadhesins) to FcRn *in vivo,* which results in an increased rate of degradation of the Fc-containing agents and, concomitantly, a reduced serum level of these agents.

The formulations disclosed herein include bulk drug compositions useful in the manufacture of pharmaceutical compositions (*e.g*., compositions that are suitable for administration to a subject or patient) which can be used in the preparation of unit dosage forms. In an embodiment, a composition of the invention is a pharmaceutical composition. Such compositions comprise a prophylactically or therapeutically effective amount of one or more prophylactic or therapeutic agents (*e.g.,* an FcRn antagonist) of the invention (or other prophylactic or therapeutic agent), and a pharmaceutically acceptable carrier.

In some embodiments the pharmaceutical compositions are formulated for administration to a subject via any suitable route of administration including, but not limited to, intramuscular, intravenous, intradermal, intraperitoneal, subcutaneous, epidural, nasal, oral, rectal, topical, inhalation, buccal (*e.g*., sublingual), and transdermal administration. In an embodiment, the pharmaceutical compositions are formulated to be suitable for intravenous administration to a subject. In an embodiment, the pharmaceutical compositions are formulated to be suitable for subcutaneous administration to a subject.

### Methods of Treatment

In an aspect, the disclosure provides methods for treating an antibody-mediated disorder (*e.g*., an autoantibody-mediated disorder) in a subject comprising administering to the subject an effective amount of an FcRn antagonist according to the disclosure or a pharmaceutical composition comprising the same. In some embodiments, the antibody-mediated disorder is an autoimmune disease.

In an embodiment, the FcRn antagonist does not antagonize FcRn binding to albumin.

In an aspect, the disclosure provides methods of reducing serum IgG in a subject comprising administering to the subject an effective amount of an FcRn antagonist according to the disclosure or a pharmaceutical composition comprising the same. In an embodiment, at least one of the IgG subtypes is reduced in a subject following administration of the FcRn antagonist. In some embodiments, IgG1, IgG2, IgG3, IgG4, or any combination thereof is reduced. In an embodiment, the level of serum IgG is decreased in the subject following administration of the FcRn antagonist compared to a baseline level of serum IgG.

In an embodiment, a total serum IgG reduction of at least about 40% compared to baseline serum IgG level is obtained. In an embodiment, a total serum IgG reduction of at least about 45% compared to baseline serum IgG level is obtained. In an embodiment, a total serum IgG reduction of at least about 50% compared to baseline serum IgG level is obtained. In an embodiment, a total serum IgG reduction of at least about 55% compared to baseline serum IgG level is obtained. In an embodiment, a total serum IgG reduction of at least about 60% compared to baseline serum IgG level is obtained. In an embodiment, a total serum IgG reduction of at least about 65%, about 70%, about 75%, or about 80% compared to baseline serum IgG level is obtained. In an embodiment, a total serum IgG reduction of at least about 65% compared to baseline serum IgG level is obtained. In an embodiment, a total serum IgG reduction of at least about 70% compared to baseline serum IgG level is obtained. In an embodiment, a total serum IgG reduction of at least about 75% compared to baseline serum IgG level is obtained. In an embodiment, a total serum IgG reduction of at least about 80% compared to baseline serum IgG level is obtained.

In an embodiment, the level of serum IgG is decreased in the subject following administration of the FcRn antagonist compared to a baseline level of serum IgG. In an embodiment, a total serum IgG reduction of about 40% compared to baseline serum IgG level is obtained. In an embodiment, a total serum IgG reduction of about 45% compared to baseline serum IgG level is obtained. In an embodiment, a total serum IgG reduction of at about 50% compared to baseline serum IgG level is obtained. In an embodiment, a total serum IgG reduction of about 55% compared to baseline serum IgG level is obtained. In an embodiment, a total serum IgG reduction of about 60% compared to baseline serum IgG level is obtained. In an embodiment, a total serum IgG reduction of about 65%, about 70%, about 75%, or about 80% compared to baseline serum IgG level is obtained. In an embodiment, a total serum IgG reduction of about 65% compared to baseline serum IgG level is obtained. In an embodiment, a total serum IgG reduction of about 70% compared to baseline serum IgG level is obtained. In an embodiment, a total serum IgG reduction of about 75% compared to baseline serum IgG level is obtained. In an embodiment, a total serum IgG reduction of about 80% compared to baseline serum IgG level is obtained.

In an embodiment, the level of FcRn is not decreased in the subject following administration of the FcRn antagonist compared to a baseline level of FcRn. In an embodiment, an FcRn reduction of less than about 1%, 2%, 3%, 4%, or 5% compared to baseline FcRn level is observed. In an embodiment, an FcRn reduction of less than about 10% compared to baseline FcRn level is observed.

In an embodiment, the level of albumin is not decreased in the subject following administration of the FcRn antagonist compared to a baseline level of albumin. In an embodiment, an albumin reduction of less than about 1%, 2%, 3%, 4%, or 5% compared to baseline albumin level is observed. In an embodiment, an albumin reduction of less than about 10% compared to baseline albumin level is observed.

In an embodiment, the total IgG, FcRn antagonist, FcRn, or albumin in a serum sample of the patient is analyzed using a bioanalytical method. In an embodiment, the total IgG, FcRn antagonist, FcRn, or albumin in a serum sample of the patient is analyzed using ELISA or automated diagnostic analyzer (IVD). In an embodiment, the total IgG, FcRn antagonist, FcRn, or albumin in a serum sample of the patient is analyzed using ELISA. In an embodiment, the total IgG, FcRn antagonist, FcRn, or albumin in a serum sample of the patient is analyzed using automated diagnostic analyzer (IVD). In an embodiment, the total FcRn in a blood sample of the patient is analyzed using a bioanalytical method, preferably flow cytometry, microscopy, or an immunoblot.

In some embodiments, the reduction of total serum IgG is measured by area under the percentage of reduction curve (AUEC). In some embodiments, the reduction of total serum IgG is measured by clearance of total serum IgG (CL).

In some embodiments, clearance of total serum IgG is increased in a subject following administration of the FcRn antagonist. In some embodiments, clearance of total serum IgG in a subject following a single therapeutic administration of the FcRn antagonist is comparable to the clearance of total serum IgG in a subject following a single therapeutic administration of efgartigimod. In some embodiments, clearance of total serum IgG in a subject following a single therapeutic administration of the FcRn antagonist is similar or the same as the clearance of total serum IgG in a subject following a single therapeutic administration of efgartigimod. In some embodiments, clearance of total serum IgG is increased in a subject following a single therapeutic administration of the FcRn antagonist compared to clearance of total serum IgG following a single therapeutic administration of efgartigimod. In some embodiments, clearance of total serum IgG is increased by at least 5%, at least 10%, at least 15%, at least 20%, at least 25%, at least 30%, at least 40%, at least 50%, at least 60%, at least 70%, at least 80%, at least 90%, at least 100%, at least 125%, at least 150%, or at least 200% in a subject following a single therapeutic administration of the FcRn antagonist compared to clearance of total serum IgG following a single therapeutic administration of efgartigimod.

In some embodiments, clearance of total serum IgG in a subject following a single administration of the FcRn antagonist is comparable to the clearance of total serum IgG in a subject following a single administration of an equivalent amount of efgartigimod. In some embodiments, clearance of total serum IgG in a subject following a single administration of the FcRn antagonist is similar or the same as the clearance of total serum IgG in a subject following a single administration of an equivalent amount of efgartigimod. In some embodiments, clearance of total serum IgG is increased in a subject following a single administration of the FcRn antagonist compared to clearance of total serum IgG following a single administration of an equivalent amount of efgartigimod. In some embodiments, clearance of total serum IgG is increased by at least 5%, at least 10%, at least 15%, at least 20%, at least 25%, at least 30%, at least 40%, at least 50%, at least 60%, at least 70%, at least 80%, at least 90%, at least 100%, at least 125%, at least 150%, or at least 200% in a subject following a single administration of the FcRn antagonist compared to clearance of total serum IgG following a single administration of an equivalent amount of efgartigimod.

In an aspect, the disclosure provides methods of enhancing intracellular uptake of IgG in myeloid cells in a subject comprising administering to the subject an effective amount of an FcRn antagonist according to the disclosure or a pharmaceutical composition comprising the same. In some embodiments, the IgG is IgG1, IgG2, IgG3, IgG4, or any combination thereof. In an embodiment, intracellular uptake of IgG is increased in the subject following administration of the FcRn antagonist compared to a baseline level of intracellular uptake of IgG.

In an embodiment, intracellular uptake of IgG is increased by at least about 5% compared to a baseline level of intracellular uptake of IgG. In some embodiments, intracellular uptake of IgG is increased by at least about 10%, at least about 15%, at least about 20%, at least about 25%, at least about 30%, at least about 35%, at least about 40%, at least about 50%, at least about 60%, at least about 70%, at least about 80%, at least about 90%, at least about 100%, at least about 125%, at least about 150%, at least about 200%, at least about 250%, or at least about 300% compared to a baseline level of intracellular uptake of IgG.

In an embodiment, intracellular uptake of IgG is increased by at least 5% compared to a baseline level of intracellular uptake of IgG. In some embodiments, intracellular uptake of IgG is increased by at least 10%, at least 15%, at least 20%, at least 25%, at least 30%, at least 35%, at least 40%, at least 50%, at least 60%, at least 70%, at least 80%, at least 90%, at least 100%, at least 125%, at least 150%, at least 200%, at least 250%, or at least 300% compared to a baseline level of intracellular uptake of IgG.

In some embodiments, the increase in intracellular uptake of IgG is measured by flow cytometry or image-based analysis.

In some embodiments, intracellular uptake of IgG in a subject following a single therapeutic administration of the FcRn antagonist is comparable to intracellular uptake of IgG in a subject following a single therapeutic administration of efgartigimod. In some embodiments, intracellular uptake of IgG in a subject following a single therapeutic administration of the FcRn antagonist is similar or the same as intracellular uptake of IgG in a subject following a single therapeutic administration of efgartigimod. In some embodiments, intracellular uptake of IgG is increased in a subject following a single therapeutic administration of the FcRn antagonist compared to intracellular uptake of IgG following a single therapeutic administration of efgartigimod. In some embodiments, intracellular uptake of IgG is increased by at least 5%, at least 10%, at least 15%, at least 20%, at least 25%, at least 30%, at least 40%, at least 50%, at least 60%, at least 70%, at least 80%, at least 90%, at least 100%, at least 125%, at least 150%, or at least 200% in a subject following a single therapeutic administration of the FcRn antagonist compared to intracellular uptake of IgG following a single therapeutic administration of efgartigimod.

In some embodiments, intracellular uptake of IgG in a subject following a single administration of the FcRn antagonist is comparable to intracellular uptake of IgG in a subject following a single administration of an equivalent amount of efgartigimod. In some embodiments, intracellular uptake of IgG in a subject following a single administration of the FcRn antagonist is similar or the same as intracellular uptake of IgG in a subject following a single administration of an equivalent amount of efgartigimod. In some embodiments, intracellular uptake of IgG is increased in a subject following a single administration of the FcRn antagonist compared to intracellular uptake of IgG following a single administration of an equivalent amount of efgartigimod. In some embodiments, intracellular uptake of IgG is increased by at least 5%, at least 10%, at least 15%, at least 20%, at least 25%, at least 30%, at least 40%, at least 50%, at least 60%, at least 70%, at least 80%, at least 90%, at least 100%, at least 125%, at least 150%, or at least 200% in a subject following a single administration of the FcRn antagonist compared to intracellular uptake of IgG following a single administration of an equivalent amount of efgartigimod.

In an embodiment, the FcRn antagonist is administered to the subject simultaneously or sequentially with an additional therapeutic agent. In an embodiment, the additional therapeutic agent is an anti-inflammatory agent. In an embodiment, the additional therapeutic agent is a corticosteroid. In an embodiment, the additional therapeutic agent is rituximab, daclizumab, basiliximab, muromonab-CD3, infliximab, adalimumab, omalizumab, efalizumab, natalizumab, tocilizumab, eculizumab, golimumab, canakinumab, ustekinumab, or belimumab. In an embodiment, the additional therapeutic agent is a leucocyte depleting agent.

In an embodiment, the additional therapeutic agent is a B-cell depleting agent. In an embodiment, the B-cell depleting agent is an antibody. In an embodiment, the B-cell depleting antibody is an antibody that specifically binds to CD10, CD19, CD20, CD21, CD22, CD23, CD24, CD37, CD53, CD70, CD72, CD74, CD75, CD77, CD79a, CD79b, CD80, CD81, CD82, CD83, CD84, CD85, or CD86.

In some embodiments, the FcRn antagonist is administered intravenously. In some embodiments, the FcRn antagonist is administered intravenously once weekly, once every two weeks, once every three weeks, once every four weeks, once monthly, or once every six weeks.

In some embodiments, the FcRn antagonist is administered subcutaneously. In some embodiments, the FcRn antagonist is administered subcutaneously once weekly, once every two weeks, once every three weeks, once every four weeks, once monthly, or once every six weeks.

### EXAMPLES

The following examples are offered by way of illustration, and not by way of limitation.

### Example 1: Human FcyRI and FcyRIIa mediates IgG internalization and delivery to FcRn

This study was performed to explore the role of FcyRs in "FcRn-mediated" recycling of IgG in IgG Fc receptor (FcR) expressing cells. Myeloid cells express several types of IgG-Fc receptors, some mostly on the surface, others intracellularly. This includes the Fc gamma receptor (FcyR) family that mediate cellular effector functions upon receptor cross-linking on the cell surface by IgG immune complexes (IC). The neonatal Fc receptor (FcRn) is almost exclusively expressed in intracellular vesicles and known for facilitating the long half-life and transcellular transport of IgG. In recent years, different groups have reported evidence for cooperative mechanisms of FcyRs and FcRn in the context of myeloid cell activation upon IgG IC binding and processing of IgG opsonized targets. This cooperative mechanism between the two Fc receptor systems could be exploited to improve delivery of engineered Fc-based therapeutics to FcRn.

### Materials and Methods

### Cells

Human Embryonic Kidney (HEK) 293F cells were obtained from Thermo Fisher Scientific and were cultured according to manufacturer's instructions. HEK-FcRn-GFP cells were generated in-house and cultured as described previously (Brinkhaus et al., Nat. Commun. 13:1-14, 2022).

THP-1 cells (ATCC TIB-202) were purchased from American Tissue Culture Collection (ATCC) and cultured according to manufacturer's instructions. In short, cells were kept in RPMI1640 (Gibco) supplemented with 10% (v/v) FCS, 100 U/ mL penicillin and 100 µg/ mL streptomycin (Thermo Fisher Scientific) at 37°C and 5% CO2 at a cell concentration between 0.2 and 0.8*10⁶ cells/mL.

Blood monocytes were isolated from freshly drawn and heparinized blood following the same procedures as previously described (Brinkhaus et al., J. Immunol. 205:1-12, 2020), using CD14+ MACS magnetic microbeads (Miltenyi Biotec). After isolation, cells were washed once with 10 mL IMDM (Gibco), supplemented with 10% (v/v) FCS and kept in the same medium.

### Generation of a THP-1 FcγRIIa K.O. cell line

THP-1 cells lacking FcyRIIa expression were generated with methods as described previously (Verhagen et al., bioRxiv 6, 2021). In short, spCas9-3xNLS was produced in BI21 star cells (Thermo Fisher Scientific) and purified by His-tag purification and size exclusion chromatography, aliquoted and frozen at -80°C. RNA guide sequences targeting exon 2 (TGGAGCACGTTGATCCACGG; SEQ ID NO: 26) and exon 3 (TTGGCAGACTCCCCATACCT; SEQ ID NO: 27) of human FcyRIIa were designed using CRISPOR and ordered at Integrated DNA Technologies (IDT). Equimolar amounts of crRNA and tracerRNA were incubated at 95°C for 5 min and cooled down at room temperature (RT) for at least 20 min under RNase-free conditions. Ribonucleoparticle (RNP) complexes were prepared by combining 7 µg of sgRNAs with 15 µg spCas9 in 30% (v/v) glycerol to a total volume of 12.3 µL. The mixture was incubated for 10 min at RT. THP-1s were passaged 2 days before nucleofection to reach a cell concentration of approximately 0.4*10⁵ cells/ mL at the day of electroporation. 1*10⁶ cells were spun down at 90 × g for 10 min at RT. Subsequently, the medium was decanted, the cells pelleted again under the same conditions for 1 min and residual medium was removed. Cells were resuspended in 100 µL of nucleofection solution, gently mixed with RNPs and transferred to nucleofector cuvette (Lonza). Electroporation was performed using program FF-100 of a 4D-Nucleofector X Unit (Lonza). After nucleofection, 1 mL of prewarmed medium was added, cells were transferred to a 15 mL tube and spun down for 5 min at 400 × g at RT. After removing the supernatant, cells were resuspended in 1.5 mL of prewarmed culture medium and expanded in a 12-well plate at 37°C at 5% CO₂. After 24 hours, the cells were spun down for 5 min at 100 × g, the supernatant was discarded, and cells were resuspended in fresh medium. 96 hours after electroporation, FcyRIIa expression was checked in flow cytometry by mouse anti-human FcyRIIa (AT10, Biorad). FcyRIIa negative population was sorted, cells expanded and cryo-preserved following standard procedures in liquid nitrogen.

### Cloning and production of soluble human FcRn and anti-biotin antibodies

Soluble human FcRn (sFcRn) was cloned, produced, and purified as described previously. In short, linear DNA strands encoding the FcRn alpha chain (fcgrt) with a C-terminal BirA deca-His -tag and β2-microglobulin (B2M) were ordered at IDT and cloned into a pcDNA3.1 expression vector. After sequence confirmation, HEK293F cells were transfected with equimolar amounts of both vectors and purified following a previously described His-purification protocol. Similarly, linear DNA strands encoding mutated Fc regions of IGHG1*3 (WT; G236A; ΔG236; M252Y S254T T256E H433K and N434F (MST-HN); G236A-MST-HN; ΔG236-MST-HN; I253A H310A H435A (IHH); and ΔG236-IHH) were ordered at IDT and cloned into pcDNA3.1 expression vectors containing an anti-biotin VH region (Kohen et al., Methods Enzymol. 279:451-463, 1997). HEK293F cells were transfected following standard procedures. Both IHH-containing IgG variants were purified using a 5 mL protein G column (GE Healthcare); all other variants were purified using protein A column (GE Healthcare). All protein purification were performed using an AKTA Prime system (GE Healthcare). Production and qualify control experiments of IgG1-Fc fragments and the anti-FcRn Fab were outsourced to Evitria AG (Switzerland). Evitria tested all molecules for size and integrity in SDS-PAGE and HPLC-SEC (≥ 98% monomeric), respectively. Once received, molecules were aliquoted and frozen at -20°C, and after thawing kept at 4°C and used for a maximum of one week.

### HPLC-SEC

All full-size anti-biotin IgG1-Fc variants and Fc fragments used for this study were tested for their monomericity in HPLC-SEC using a 10/300 GL Superdex 200 column (GE Healthcare).

### SPR

Affinity measurements to human FcRn and human FcyRs were performed using an IBIS MX96 (IBIS Technologies) device and a Continuous Flow Microspotter (Wasatch Microfluidics). In short, for FcRn affinity measurements, IgG-Fc containing molecules were spotted at 4 concentrations in a 2-fold dilution series starting at 60 nM on a SensEye G Easy2Spot (SensEye) in 10 mM sodium acetate at pH 4.5 supplemented with 0.075% (v/v) Tween (80). Human or mouse sFcRn was injected at 12 concentrations starting from 0.49 nM to 1000 nM (2-fold dilution series) in 1xPBS (Fresenius Kabi) containing 0.075% (v/v) Tween (80). Independent experiments were performed to determine affinities at pH 7.4 and 6.0. The sensor surface was regenerated between the cycles by subsequent injections of 20 mM Tris-HCl, 150 mM NaCl pH 8.8 and 20 mM H₃PO₄ pH 2.4. For FcyR affinity measurements, an array of 4 concentrations of a 3-fold dilution series of C-terminally biotinylated human FcyRs was captured simultaneously on a SensEye G-streptavidin sensor in 1xPBS containing 0.075% (v/v) Tween (80). The highest concentrations differed depending on the receptor and was 100 nM for hFcyRIIIa-158V (Sino Biological), 30 nM for hFcyRIIa-131H (Sino Biological), hFcyRIIIa-158F (Sino Biological), hFcγRIIIb-NA1 (in-house) and hFcyRIIIb-NA2 (in-house) and 10 nM for hFcyRIIa-131R (Sino Biological) and hFcyRIIb (Sino Biological), respectively. A 2-fold dilution series of IgG variants or IgG-Fc fragments thereof was injected ranging from 0.49 nM to 1000 nM in 1xPBS at pH 7.4 supplemented with 0.075% (v/v) Tween (80). In separate experiments, 4 concentrations of a 3-fold dilutions series of C-terminally biotinylated anti-His mIgG1 (GenScript) was captured on a SensEye G-streptavidin sensor in 1xPBS containing 0.075% (v/v) Tween (80). Subsequently, 25 nM of C-terminally His-tagged hFcyRIa (Sino Biologicals) was captured and IgG variants or IgG-Fc fragments thereof were titrated from 1.4 nM to 100 nM in a 2-fold dilution series in 1xPBS containing 0.075% (v/v) Tween (80). For FcyR affinity measurements, similar experiments with the full-size IgG variants were performed in 1xPBS containing 0.075% (v/v) Tween (80) and 0.1% (m/v) BSA (Thermo Fisher Scientific) at pH 6.0. Regeneration of the sensor surface between the cycles was performed by injection of 10 mM Gly-HCl pH 2.4. Affinity calculations were carried out by performing an equilibrium analysis interpolating to an Rmax of 200 to 600, depending on the assay, and fitting a 1:1 Langmuir binding model, assuming equilibrium to be reached after 360s of IgG-Fc containing analyte injections. Analysis and calculations were performed using Scrubber Software Version 2 (BioLogic Software), Excel (Microsoft) and Graphpad Prism (Prism).

### Validation experiments to assess intracellular accumulation

2×10⁵ THP-1 cells were loaded for 2h with IgG-Fc variants at 100 nM in a total volume of 200 µL of RPMI1640 (Gibco) supplemented with 10% (v/v) FCS, 100 U/ mL penicillin and 100 µg/ mL streptomycin (Thermo Fisher Scientific) per well at 37°C and 5% CO₂. Cells were washed five times with 150 µL ice-cold medium as described above, followed by centrifugation at 450 × g at 4°C for 3 min and removal of the supernatant. Cells were then washed once with 150 µL 1xPBS supplemented with 1% (v/v) FCS and 0.2M Gly-HCl at pH 3.0 (stripping buffer) to remove surface bound IgG. Independent samples of 50 µL per well were taken after loading, after five times washing with medium and after washing with stripping buffer and were transferred to 150 µL of ice cold 1xPBS with 1% (v/v) FCS. Cells were spun down at 450 × g at 4°C for 3 min and washed once more with 150 µL of ice cold 1xPBS containing 1% (v/v) FCS. Cells were stained in 50 µL of 1/1000 mouse anti-human Fc-FITC (Clone JDC-10, Southern Biotec) and 1/1000 LIVE/DEAD Fixable Near-IR Dead Cell Stain Kit (Thermo Fisher Scientific) for 30 min in ice cold 1xPBS with 1% (v/v) FCS on ice. Subsequently, cells were washed twice with 150 µL and then resuspended in 50 µL of ice cold 1xPBS with 1% (v/v) FCS and measured in flow cytometry.

### Labeling of IgG Fc fragments

IgG-Fc fragments were labeled with Dylight (DL) 405 (Thermo Fisher Scientific) according to manufacturer's instructions and as described previously. In short, all Fc fragments were buffer exchanged to 1xPBS (Fresenius Kabi) using Amicon Ultra 0.5 mL Centrifugal Filters (MWCO 10kDa) (Amicon) and adjusted to the same concentration. A master mix for the labeling was prepared, aiming for a final concentration of 0.1 M NaHCO₃ (Thermo Fisher Scientific) in 1xPBS and a molar ratio of 15:1 (Dylight405 NHS:IgG1-Fc variant) per reaction. The mixtures were vortexed shortly, spun down and incubated for 2h at RT. Free dye was removed using Amicon Ultra 0.5 mL Centrifugal Filters (MWCO 10kDa) (Amicon). An anti-FcRn Fab and a human IgG Fab isotype control (Bio-Techne) were labeled with Dylight 650 following the same procedure, using a molar ratio of 5:1 (Dylight650 NHS:anti-FcRn Fab). Degree of labeling (DoL) was determined by UV/Vis using a Nanodrop 2000 (Thermo Fisher Scientific) according to manufacturer's protocol.

### Intracellular accumulation after removal of surface-bound IgG by flow cytometry

1×10⁵ THP-1 or THP-1 FcyRIIa K.O. cells were loaded for 2h with DL405-labeled IgG-Fc variants at 100 nM in a total volume of 100 µL of RPMI1640 (Gibco) supplemented with 10% (v/v) FCS, 100 U/ mL penicillin and 100 µg/ mL streptomycin (Thermo Fisher Scientific) per well at 37°C and 5% CO₂. The assay was performed in duplicate per condition in a sterile 96-well U-bottom plate. Cells were subsequently washed five times with 150 µL ice cold culture medium as used for loading the cells. Cells were subsequently washed once with ice cold stripping buffer (1×PBS supplemented with 1% (v/v) FCS and 0.2M Gly-HCl at pH 3.0), followed by two washes with ice cold 1xPBS with 1% (v/v) FCS, while spinning down the cells at 450 x g for 3 min at 4°C and discarding the supernatant in between, as described above. Cells were resuspended in 50 µL of the same buffer, transferred to Micronics tubes (Micronic) and measured in flow cytometry.

### Intracellular accumulation by Image Stream

2×10⁵ THP-1 or THP-1 FcyRIIa K.O. cells were loaded for 2h with DL405-labeled IgG-Fc variants at 100 nM in a total volume of 200 µL of RPMI1640 (Gibco) supplemented with 10% (v/v) FCS, 100 U/ mL penicillin and 100 µg/ mL streptomycin (Thermo Fisher Scientific) per well in a sterile U-bottom plate (Corning) at 37°C and 5% CO₂. Cells were washed twice with 150 µL of ice cold 1xPBS supplemented with 1% (v/v) FCS at pH 7.4, followed by centrifugation at 450 x g for 3 min at 4°C and stained in 50 µL with 1/20 mouse anti-human CD33-PE (clone P67.7, BD Biosciences) and 1/20 mouse anti-human CD45 FITC (clone 2D1, BD Biosciences) in 50 µL of 1×PBS supplemented with 1% (v/v) FCS at pH 7.4 on ice in the dark. Cells were then washed twice with 150 µL 1xPBS containing 1% (v/v) FCS at pH 7.4, resuspended in 50 µL of the same buffer, transferred to 1.5 mL Eppendorf Tubes (Eppendorf) on ice and measured in image stream. At least 2000 events were measured in the live gate.

### Intracellular FcRn occupancy assay

The experiments were performed as described previously (Brinkhaus et al., Nat. Commun. 13:1-14, 2022). In short, 1×10⁵ cells were loaded with 5-fold dilution series of IgG variants and fragments thereof for 2h, ranging from 4 to 500 nM under culture conditions, as specified above. Non-titrated negative control (IgG1-Fc-IHH) was tested at 500 nM only. When indicated, cells were preloaded for 2h with 1.125 µg/mL IVIg (Nanogam, Sanquin) in a total volume of 100 µL per well. 95 µL of this cell solution was then combined with 5 µL of 20x concentrated dilution series of IgG variants and incubated for another 2h under culture conditions. Thereafter, cells were transferred to 96-well V-bottom plate (Corning) and incubated on ice for 2 min. Cells were subsequently spun down for 3 min at 450 x g at 4°C and the supernatant was discarded. Following the same procedure, cells were washed five times with 150 µL ice-cold medium, followed by one wash with 150 µL 1xPBS supplemented with 1% (v/v) FCS at pH 7.4 and one wash with 150 µL of 1xPBS supplemented with 1% (v/v) FCS at pH 6.0. Cells were then fixed and permeabilized using both solutions of a Fixation/Permeabilization Kit (BD Biosciences) adapted to pH 6.0 after addition of MES (Thermo Fisher Scientific) to a final concentration of 0.5M, otherwise following manufacturer's instructions. Staining for available FcRn was performed using 1 µg/mL of DL 650 (Thermo Fisher Scientific) labeled anti-FcRn Fab in Cytoperm solution (0.5M MES, pH 6.0) of the Kit (BD Biosciences) described above, for 30 min on ice in the dark. Cells were washed twice with 150 µL 1xPBS supplemented with 1% (v/v) FCS at pH 6.0, spun down at 450 x g at 4 °C for 3 min and the supernatants were discarded. Finally, cells were resuspended in 50 µL of the same buffer, transferred to Micronics tubes (Micronic) on ice and measured in flow cytometry.

### Flow cytometry and Image stream experiments

Flow cytometry measurements were performed using a BD LSR-II (BD Biosciences) and a BD Fortessa Cell Analyzer (BD Biosciences) using BD FACSDiva Software (BD Biosciences). 10,000 events were measured in the gate of interest if not indicated differently. Cell sorting to create the THP-1 FcyRIIa K.O. cells was performed using a BD FACSAria II cell sorter (BD Biosciences). Analysis was performed using FlowJo 10.7.1 Software (BD Biosciences). Image cytometry was performed using an Amnis Image Stream MkII (Amnis) and INSPIRE software (version 200.1.681.0) was used to obtain the data. 2000 events per condition in the live gate were measured. Analysis was performed using IDEAS 6.2 (Amnis).

### Statistical analysis and data presentation

Graphpad Prism 8 was used for data presentation and statistical analysis. Depending on the assay, statistical analyses were performed using a one-way ANOVA (Holm-Sidak's multiple-comparisons test) comparing to IgG1-WT (ImageStream experiments), 2-way ANOVA (Sidak's multiple-comparisons test) comparing all groups (flow cytometry experiments) or 2-way ANOVA (Dunnett's multiple-comparisons test) comparing to Fc-MST-HN (flow cytometry experiments).

### Results

### Design of IgG molecules with modified FcyR and FcRn binding properties

IgG1-Fc fragments were designed, combining previously described amino acid substitutions in the IgG Fc region, with the aim of obtaining Fc molecules with different FcyR and FcRn binding properties. FcyRIIa binding was enhanced specifically through the G236A substitution, and all FcyR binding abrogated using ΔG236. FcRn binding was enhanced using the MST-HN substitutions, and FcRn binding abrogated using the IHH substitutions. The substitutions at position 236 described above were also combined with the mutations that modify FcRn binding.

### IgG molecules and Fc fragments show expected FcyR and FcRn binding properties

Full-length anti-biotin IgG molecules and Fc-only fragments used in this study were tested in HPLC-SEC and were found to have the expected size and were ≥ 98% monomeric. Production and quality control of all antibody fragments were outsourced to Evitria (Switzerland) and fulfilled the same criteria as described above.

Binding properties of the Fc fragments were analyzed with human FcyRs and human FcRn coupled through a C-terminal biotin to streptavidin multiplex SPR arrays. Sensorgrams of a representative of three independent affinity measurements were used to determine the binding of the human IgG1-Fc variants to human FcyRs and human FcRn at pH 7.4 and pH 6.0. The array of FcyRs was immobilized at 4 different concentrations and human IgG1-Fc fragments were titrated in a 2-fold dilution series from 1000 nM to 0.49 nM. For affinity measurements to human FcRn, 4 concentrations of each of the Fc variants were immobilized on the sensor and human FcRn was titrated in a 2-fold dilution series from 1000 nM to 0.49 nM. K_{D} values were derived by performing an equilibrium analysis and fitting a 1:1 Langmuir binding model. When binding was observed, but no affinity could be calculated, it was labeled as > 2000 nM. No binding was marked as non-determinable (n.d.). Data are presented in **Tables S1-S4** as mean ± standard deviation.

Overall, the molecules bound FcyRs as expected **(Table S1).** Fc-ΔG236 showed no binding to any of the FcyRs tested, also not when combined with the MST-HN mutations. Fc-G236A was found to exhibit specifically increased binding to both FcyRIIa variants by 4-fold compared to Fc-WT (wild-type), whereas binding to all other FcyRs was reduced up to 2-fold. Although structurally distinct, both sets of mutations modifying FcRn binding, MST-HN and IHH, led to a reduction of binding to all low affinity FcyRs by a factor of at least 3, as previously reported. This MST-HN-related reduction in affinity was also seen when combined with G236A, resulting in a 2-fold increase in FcyRIIa binding compared to Fc-WT. However, Fc-G236A-MST-HN exhibited an approximately 9- and 6-fold increase in affinity compared to Fc-MST-HN to FcyRIIa-131H and -131R, respectively.

The binding affinities of full-length IgG1 antibodies comprising the same sets of Fc mutations to human FcyRs at pH 7.4 was similar to the binding affinities of the Fc fragments to human FcyRs at pH 7.4 **(Table S2).** Additionally, the affinities of the full-length IgGs to FcyRs at pH 6.0 were measured and only FcyRI binding was reduced about 3- to 4-fold compared to FcyRI binding at pH 7.4 **(Table S3).**

Generally, there was no impact of the FcyR binding modifying mutations on binding of the Fc fragments to FcRn **(Table S4).** The relative FcRn affinities of all non-MST-HN- and all MST-HN-containing variants were comparable, respectively. Of all variants assayed, only the MST-HN variants exhibited binding at pH 7.4 and showed increased affinity compared to Fc-WT at pH 6.0, in line with previous findings. IHH variants did not bind FcRn at pH 7.4 or pH 6.0.

**Table S1: Binding affinity (K_{D}) of human Fc fragment variants to human FcyRs**

| | **FcγRIa** | **FcγRIIa-131H** | **FcγRIIa-131R** | **FcγRII b** | **FcγRIIIa -158F** | **FcγRIIIa -158V** | **FcγRIII b- NA1** | **FcγRIII b-NA2** |
|---|---|---|---|---|---|---|---|---|
| **Fc-WT** | 4.4 ± 1.4 nM | 404.5 ± 36.2 nM | 580.2 ± 20.6 nM | >2000 nM | 481.5 ± 14.4 nM | 151.3 ± 12.0 nM | >2000 nM | >2000 nM |
| **Fc-G236A** | 7.1 ± 1.7 nM | 91.1 ± 10.7 nM | 165.8 ± 13.1 nM | >2000 nM | 809.6 ± 56.2 nM | 310.8 ± 27.0 nM | >2000 nM | >2000 nM |
| **Fc-ΔG236** | n.d. | n.d. | n.d. | n.d. | n.d. | n.d. | n.d. | n.d. |
| **Fc-IHH** | 6.1 ± 1.8 nM | >2000 nM | >2000 nM | >2000 nM | >2000 nM | 488.5 ± 52.9 nM | >2000 nM | >2000 nM |
| **Fc-MST-HN** | 5.9 ± 2.2 nM | 1648.2 ± 135.7 nM | 1636.6 ± 197.3 nM | >2000 nM | 1769.7 ± 181.3 nM | 417.8 ± 44.4 nM | >2000 nM | >2000 nM |
| **Fc-G236A-MST-HN** | 10.8 ± 2.8 nM | 189.3 ± 23.3 nM | 268.7 ± 23.1 nM | >2000 nM | >2000 nM | 859.9 ± 119.2 nM | >2000 nM | >2000 nM |
| **Fc-ΔG236-MST-HN** | n.d. | n.d. | n.d. | n.d. | n.d. | n.d. | n.d. | n.d. |
| **Fc-ΔG236-IHH** | n.d. | n.d. | n.d. | n.d. | n.d. | n.d. | n.d. | n.d. |

**Table S2: Binding affinity (K_{D}) of full-length human anti-biotin-IgG1 variants to human FcyRs at pH 7.4**

| | **FcγRI a** | **Fc γRIIIa-131H** | **FcγRIIa -131R** | **FcγRII b** | **FcγRIIIa -158F** | **FcγRIIIa -158V** | **FcγRIIIb - NA1** | **FcγRIIIb -NA2** |
|---|---|---|---|---|---|---|---|---|
| **IgG1-WT** | 7.1 ± 0.6 nM | 668.3 ± 92.5 nM | 952.9 ± 233.1 nM | >2000 nM | >2000 nM | 382.2 ± 47.5 nM | >2000 nM | >2000 nM |
| **IgG1-MST-HN** | 7.5 ± 1.0 nM | >2000 nM | >2000 nM | >2000 nM | >2000 nM | 1017.5 ± 151.5 nM | >2000 nM | >2000 nM |
| **IgG1-ΔG236** | >2000 nM | n.d. | n.d. | n.d. | n.d. | n.d. | n.d. | n.d. |
| **IgG1-ΔG236-MST-HN** | n.d. | n.d. | n.d. | n.d. | n.d. | n.d. | n.d. | n.d. |
| **IgG1-IHH** | 9.1 ± 1.3 nM | >2000 nM | >2000 nM | >2000 nM | >2000 nM | 943.6 ± 102.6 nM | >2000 nM | >2000 nM |
| **IgG1-ΔG236-IHH** | n.d. | n.d. | n.d. | n.d. | n.d. | n.d. | n.d. | n.d. |
| **IgG1-G236A** | 13.2 ± 0.8 nM | 125.7 ± 4.4 nM | 240.9 ± 6.8 nM | >2000 nM | >2000 nM | >2000 nM | >2000 nM | n.d. |
| **IgG1-G236A-MST-HN** | 14.8 ± 2.1 nM | 288.5 ±62.4 nM | 401.5 ± 85.6 nM | >2000 nM | >2000 nM | >2000 nM | n.d. | n.d. |
| **IgG1-EPGHPA** | 32.9 ± 1.2 nM | >2000 nM | 963.9 ± 115.7 nM | 47.5 ± 3.0 nM | n.d. | n.d. | n.d. | n.d. |
| **IgG1-EPGHPA -MST-HN** | 40.2 ± 3.9 nM | >2000 nM | 1365.0 ± 304.6 nM | 53.4 ± 6.7 nM | n.d. | n.d. | n.d. | n.d. |
| **IgG1-WT-afuc** | 32.9 ± 1.2 nM | 1003.4 ± 93.1 nM | 1008.7 ± 1001 nM | >2000 nM | 80.7 ± 8.7 nM | 16.6 ± 1.7 nM | 617.0 ± 126.0 nM | 552.4 ± 144.6 nM |
| **IgG1-MST-HN-afuc** | 40.2 ± 3.9 nM | >2000 nM | >2000 nM | >2000 nM | 182.2 ± 13.2 nM | 31.7 ± 2.1 nM | >2000 nM | >2000 nM |

**Table S3: Binding affinity (K_{D}) of full-length human anti-biotin-IgG1 variants to human FcyRs at pH 6.0**

| | **FcγRI a** | **FcγRIIa -131H** | **FcγRIIa -131R** | **FcγRII b** | **FcγRIIIa -158F** | **FcγRIIIa -158V** | **FcγRIIIb - NA1** | **FcγRIIIb -NA2** |
|---|---|---|---|---|---|---|---|---|
| **IgG1-WT** | 27.0 ± 2.9 nM | 455.1 ± 143.9 nM | 920.3 ± 112.6 nM | >2000 nM | 893.9 ± 220.6 nM | 336.9 ± 95.7 nM | >2000 nM | >2000 nM |
| **IgG1-MST-HN** | 36.9 ± 6.3 nM | >2000 nM | >2000 nM | >2000 nM | 1253.2 ± 220.6 nM | 783.9 ± 106.3 nM | >2000 nM | >2000 nM |
| **IgG1-ΔG236** | >2000 nM | n.d. | n.d. | n.d. | n.d. | n.d. | n.d. | n.d. |
| **IgG1-ΔG236-MST-HN** | n.d. | n.d. | n.d. | n.d. | n.d. | n.d. | n.d. | n.d. |
| **IgG1-IHH** | 34.5 ± 13.3 nM | >2000 nM | >2000 nM | >2000 nM | 1258.3 ± 298.1 nM | 756.9 ± 101.0 nM | >2000 nM | >2000 nM |
| **IgG1-ΔG236-IHH** | n.d. | n.d. | n.d. | n.d. | n.d. | n.d. | n.d. | n.d. |
| **IgG1-G236A** | 63.3 ± 17.8 nM | 141.2 ± 19.6 nM | 358.3 ± 63.0 nM | >2000 nM | >2000 nM | 491.3 ± 45.1 nM | >2000 nM | n.d. |
| **IgG1-G236A-MST-HN** | 90.0 ± 30.0 nM | 336.2 ± 48.7 nM | 707.2 ± 90.4 nM | >2000 nM | >2000 nM | 1010.0 ± 215.3 nM | n.d. | n.d. |
| **IgG1-EPGHPA** | 37.5 ± 6.2 nM | >2000 nM | 962.6 ± 244.7 nM | 58.2 ± 16.8 nM | n.d. | n.d. | n.d. | n.d. |
| **IgG1-EPGHPA -MST-HN** | 54.6 ± 16.6 nM | >2000 nM | 1558.4 ± 116.7 nM | 68.2 ± 13.4 nM | n.d. | n.d. | n.d. | n.d. |
| **IgG1-WT-afuc** | 33.3 ± 2.7 nM | 1016.7 ± 142.3 nM | 1993.6 ± 223.8 nM | >2000 nM | 101.5 ± 30.3 nM | 17.8 ± 4.6 nM | >2000 nM | 625.3 ± 177.3 nM |
| **IgG1-MST-HN-afuc** | 35.6 ± 6.1 nM | >2000 nM | >2000 nM | >2000 nM | 204.6 ± 67.8 nM | 31.4 ± 9.8 nM | >2000 nM | 900.0 ± 224.2 nM |

**Table S4: Binding affinity (K_{D}) of human Fc fragment variants to human FcRn at pH 7.4 & 6.0**

| | **pH 7.4** | **pH 6.0** |
|---|---|---|
| **Fc-WT** | n.d. | 130.4 ± 5.1 nM |
| **Fc-G236A** | n.d. | 127.5 ± 2.3 nM |
| **Fc-ΔG236** | n.d. | 130.1 4.8 nM |
| **Fc-IHH** | n.d. | n.d. |
| **Fc-MST-HN** | 1414.0 ± 174.9 nM | 18.9 ± 1.5 nM |
| **Fc-G236A-MST-HN** | 1335.0 ± 225.2 nM | 23.4 ± 2.3 nM |
| **Fc-ΔG236-MST-HN** | 1415.6 ± 303.9 nM | 15.4 ± 0.8 nM |
| **Fc-ΔG236-IHH** | n.d. | n.d. |

**Table S5: Summary of binding affinity (K_{D}) characteristics of human Fc fragment variants to human FcyRs and FcRn**

| | **Fc-WT** | **Fc-G236A** | **Fc-ΔG236** | **Fc-IHH** | **Fc-MST-HN** | **Fc-G236A-MST-HN** | **Fc-ΔG236-MST-HN** | **Fc-ΔG236-IHH** |
|---|---|---|---|---|---|---|---|---|
| **FcγRI** | ≈ | ↓ | ↓↓ | ≈ | ≈ | ↓ | ↓↓ | ↓↓ |
| **FcγRIIa** | ≈ | ↑↑ | ↓↓ | ↓ | ↓ | ↑ | ↓↓ | ↓↓ |
| **FcRn** | ≈ | ≈ | ≈ | ↓↓ | ↑↑ | ↑↑ | ↑↑ | ↓↓ |

| | | | | | | | | |
|---|---|---|---|---|---|---|---|---|
| ≈ indicates similar binding to Fc-WT; ↓ indicates ≥ 2x decrease in binding relative to Fc-WT; ↓↓ indicates ≥ 4x decrease in binding relative to Fc-WT; ↑ indicates ≥ 2x increase in binding relative to Fc-WT; ↑↑ indicates ≥ 4x increase in binding relative to Fc-WT. | | | | | | | | |

### FcyR binding is crucial for intracellular accumulation of IgG

Next, the uptake of the Fc fragments by human monocytic THP-1 cells, which endogenously express FcyRIa, FcyRIIa and FcRn, was examined. Given that FcyRs are surface receptors and FcRn is mainly expressed intracellularly, it can be technically challenging to distinguish surface-bound from intracellular IgG with cells expressing both receptor systems. First, an Image Stream platform was used to assess intracellular localization of directly DL405-labeled IgG-Fc variants engineered with different FcyR/FcRn-binding properties (see Tables S1-S5). The cells were incubated for 2h with the Fc fragments, and the cell surface stained by two independent markers (CD33 and CD45) and the intensity of the Fc signals measured, which were not colocalized with the surface markers.

Striking patterns were observed depending on the FcyR- and FcRn-binding characteristics using both image data (data not shown) and calculated relative intensities (Table S6). Abrogating FcyR binding resulted in almost complete loss of signal intensity of intracellular Fc staining compared to Fc-WT in both non-FcRn-modified (Fc-ΔG236) and FcRn-abrogated (Fc-ΔG236-IHH) variants. In contrast, enhanced FcyRIIa binding (Fc-G236A) and enhanced FcRn binding (Fc-MST-HN) increased the signals by 1.22 ± 0.09-fold and 1.29 ± 0.1-fold, respectively. Combining both enhanced FcyRIIa and FcRn binding (Fc-G236A-MST-HN) increased the signals by 1.87 ± 0.34-fold, indicating a synergistic internalization by these receptors. Abrogating FcRn binding (Fc-IHH) led to a minor decrease in intensities to 0.92 ±0.09-fold compared to Fc-WT, indicating FcRn binding is not required for entry into these cells. Interestingly, enhanced FcRn binding in an FcyR-dead backbone (Fc-ΔG236-MST-HN) partially rescued the loss of fold-intensity observed for the other ΔG236-containing FcyR-dead variants, however showed lower signals as compared to Fc-MST-HN (0.61 ± 0.06 vs. 1.29 ± 0.1). These results indicated a role for both FcRn and FcyRs in uptake of monomeric IgG to monocytic THP-1 cells.

**Table S6: Fold-intensity of intracellular accumulation of Fc fragment variants compared to Fc-WT**

| | **Fc-WT** | **Fc-G236A** | **Fc-ΔG236** | **Fc-IHH** | **Fc-MST-HN** | **Fc-G236A-MST-HN** | **Fc-ΔG236-MST-HN** | **Fc-ΔG236-IHH** |
|---|---|---|---|---|---|---|---|---|
| **Fold-intensity of Fc-WT** | 1 | 1.22 ± 0.09* | -0.03 ± 0.11** | 0.92 ± 0.09 | 1.29 ± 0.1* | 1.87 ± 0.34* | 0.61 ± 0.06** | -0.06 ± 0.18** |

| | | | | | | | | |
|---|---|---|---|---|---|---|---|---|
| * p < 0.05; ** p < 0.01 | | | | | | | | |

To support the image-based analyses, a classical flow cytometry-based approach was used to verify these observations using brief acidic strip to remove surface bound IgG. The results obtained using this method **(****FIG. 1****)** were consistent with the results obtained using the Image Stream technique **(Table S6),** confirming that both FcRn and FcyRs can mediate uptake of monomeric IgG to monocytic THP-1 cells. Next, the role of FcyRIIa in this process was investigated by comparing the IgG-Fc uptake in WT THP-1 with IgG-Fc uptake in THP-1 cells engineered to lack FcyRIIa (FcyRIIa K.O. THP-1 cells). The uptake of all IgG-Fc variants with enhanced FcyRIIa binding (G236A) binding was reduced to levels comparable to the non-FcyR-modified counterparts, WT and MST-HN **(****FIG. 1****)** in FcyRIIa K.O. THP-1 cells. These observations imply a compensatory effect of FcyRI in the uptake of monomeric IgG, when FcyRIIa is not functionally expressed.

### Fc-MST-HN with enhanced FcγRIIa binding occupies FcRn more efficiently

To gain insight of the intracellular location following the internalization, the effect of FcyR binding on intracellular occupancy of FcRn was examined. An FcRn occupancy assay, using an anti-FcRn Fab only recognizing FcRn not in complex with Fc-MST-HN, was used as previously described. First, FcRn occupancy was measured in HEK-FcRn-GFP cells, which overexpress FcRn, but do not express FcyRs. All MST-HN Fc variants with enhanced FcRn binding showed similar dose-dependent FcRn occupancy, but not Fc-WT nor Fc-IHH **(****FIG. 2****,** panel a). In contrast, THP-1 cells, which express FcyRI and FcyRIIa and have high endogenous levels of FcRn, exhibited an FcyR-dependent FcRn occupancy pattern: abrogating FcyR binding in an Fc-MST-HN (Fc-ΔG236-MST-HN) construct led to a significantly decreased FcRn occupancy compared to Fc-MST-HN over the whole concentration range tested **(****FIG. 2****,** panel b). Importantly, FcyRIIa-enhanced Fc-G236A-MST-HN showed significantly increased FcRn occupancy, in line with the observed increased intracellular accumulation. This enhancement was lost in THP-1 FcyRIIa K.O. cells, where the defective internalization of Fc-ΔG236-MST-HN became less clear **(****FIG. 2****,** panel c). This indicated that both FcyRI and FcyRIIa are involved in delivering Fc-MST-HN to FcRn.

To dissect the influence of both receptors, FcyRI and FcyRII, experiments were performed in cells preloaded with IVIg, mimicking irrelevant IgG concentrations as found in the serum and blocking mostly the high-affinity FcyRI. Also here, the lack of FcyR binding did not significantly affect the occupancy of the Fc-ΔG236-MST-HN variant, whereas Fc-G236A-MST-HN with its enhanced FcyRIIa affinity showed significantly increased FcRn occupancy compared to both Fc-MST-HN and Fc-ΔG236-MST-HN, confirming the pivotal role of FcyRIIa in delivery of Fc-MST-HN to FcRn **(****FIG. 2****,** panel d). Consequently, when performing the assay in the presence of IVIg on THP-1 FcyRIIa K.O. cells, no differences in FcRn occupancy was observed between the MST-HN variants with modified FcyR binding **(****FIG. 2****,** panel e), comparable to HEK-FcRn cells with no FcyR expression **(****FIG. 2****,** panel a).

Next, experiments were performed with primary monocytes. Similar to results in THP-1 cells **(****FIG. 2****,** panel b), FcyRIIa-enhanced Fc-G236A-MST-HN occupied FcRn significantly better, while the Fc-ΔG236-MST-HN occupied FcRn significantly less than Fc-MST-HN **(****FIG. 2****,** panel f). Finally, the recycling capacity of the MST-HN variants was examined in THP-1 cells after a chase period replacing the cell medium without FcRn inhibitor. All Fc-MST-HN variants showed prolonged occupancy of FcRn in the same order of uptake efficiency, indicating similar recycling behavior **(****FIG. 2****,** panel g). Importantly, when performing the assay for a prolonged time, Fc-MST-HN and Fc-G236A-MST-HN exhibited increased FcRn occupancy and reached a steady state, indicating a higher degree of receptor occupancy as compared to Fc-ΔG236-MST-HN, implying re-uptake of the molecules **(****FIG. 3A****).** Results obtained from full-length IgG Fc variants were similar to those obtained from Fc fragment variants, but with overall lower FcRn occupancy **(****FIG. 3B****),** suggesting negative impact of the Fabs on intracellular occupancy consistent with previous findings.

### Conclusions

Cooperative mechanisms between FcyRs and FcRn have been reported previously, however only in the context of multimeric IgG ICs. In this study, FcyRs are shown to be required for efficient entry of monomeric IgG into myeloid cells, where they become accessible for FcRn. Enhancing the binding of an Fc-MST-HN to FcyRIIa increases cellular entry and intracellular FcRn occupancy compared to non-FcyR-enhanced or FcyR-silenced Fc-MST-HN.

This data suggest that FcyR-dependent uptake might not be restricted to multimeric IgG ICs, and may also apply to monomeric IgG. Most importantly, the results suggest that IgG lacking FcyR-binding properties do not readily enter FcyR expressing monocytic cells, and enhanced FcyR binding, enhanced FcRn binding, or preferentially both, promote cellular entry of IgG. This in turn suggests a receptor-mediated rather than a random pinocytotic mechanism to be an important entry mechanism of IgG into myeloid cells.

The effect of FcyRIa, FcyRIIa, and FcRn receptors on IgG cellular uptake were the focus of this study, given the ubiquitous expression on and in cells of the myeloid compartment considered relevant for IgG recycling. No set of Fc substitutions have been described which specifically enhances binding to FcyRIa. However, the MST-HN mutations reduce binding to all low-affinity receptors compared to WT IgG, which allowed investigation of the role of FcyRIa and FcyRIIa in delivery of Fc-MST-HN to FcRn. To specifically enhance FcyRIIa binding, Fc-G236A and Fc-G236A-MST-HN were used. Although the MST-HN mutations also negatively affected G236A-enhanced FcyRIIa binding, the variant exhibited an about 2-fold improvement compared to WT IgG and a 9- and 6-fold improvement over Fc-MST-HN at neutral pH to FcyRIIa-131H and -131R, respectively. Strikingly, all IgG variants bound FcyRs at acidic pH 6.0 comparably well as at neutral pH, except for FcyRI, which exhibited at least a 3- to 4-fold decrease in affinity (only tested for full-length anti-biotin IgG).

In this study, evidence is presented for the first time indicating that FcyR on myeloid cells are highly efficient entry points for monomeric IgG into subcellular compartments. Without efficient FcyR or FcRn crosslinking, these monomeric IgG are rescued from degradation by FcRn by recycling the IgG back to the surface, suggesting FcRn function is partly to prevent myeloid cells from functioning as IgG sinks. Entry of monomeric IgG in myeloid cells appears to be receptor-mediated rather than pinocytosis-mediated. Lastly, this data suggests that this mechanism can be exploited in vivo to improve IgG-based therapeutics, by specifically targeting specific cells or tissues for entry to exert their biological functions.

### Example 2: Pharmacokinetics/Pharmacodynamics of Fc fragment variants in humanized FcRn/FcyR mice

The current experiment was conducted to compare PK and PD of Fc fragments with enhanced FcRn and FcyRIIa binding (Fc-G236A-MST-HN) to Fc fragments with enhanced FcRn binding in which FcyR binding is eliminated (Fc-ΔG236-MST-HN) in humanized FcRn/FcyR mice.

### Methods

Briefly, a total of 30 humanized FcRn/FcyR mice were randomly assigned into 6 groups. The mice were single-dosed intravenously according to the designated group and doses in Table S7. Tracer IgG (20 mg/kg) and IVIg (200 mg/kg) were administered to all groups 3 days prior to administration of test items. All animals were pre-weighed before dosing and dosed according to their body weights. Blood samples were collected before dosing of the test article (pre-dose, d-2) and after treatment for PD, PK, and ADA read-outs for 14 days **(Table S7).**

Blood samples were processed to serum and added to a 96-well plate (polypropylene) and stored at -80°C.

**Table S7: Groups and dosing regimen**

| **Group** | **N** | **Test Article** | **Dose, mg/kg** | **Administration route** | **Blood sampling times** |
|---|---|---|---|---|---|
| A | 5 | Fc-G236A-MST-HN | 2 | IV, single dose | -2d, 0d+5m, 1d, 2d, 4d, 7d, 14d |
| B | 5 | | 5 | | |
| C | 5 | | 20 | | |
| D | 5 | Fc-ΔG236-MST-HN | 2 | | |
| E | 5 | | 5 | | |
| F | 5 | | 20 | | |

Tracer IgG1 and IVIg serum levels, concentrations of Fc fragment variants, and immune response against the test items, were determined using sandwich ELISA and were plotted over time.

### Results

To assess the PD effects of Fc fragments with enhanced FcRn and FcyRIIa binding (Fc-G236A-MST-HN) to Fc fragments with enhanced FcRn binding in which FcyR binding is eliminated (Fc-ΔG236-MST-HN) on serum IgG concentration, IVIg and tracer IgG were administered to the mice prior to treatment with the test articles. The levels of IVIg and tracer IgG were measured during the course of the study. The measured IVIg concentrations were plotted over time per treatment group **(****FIGs. 4A-4C****).**

As expected, a clear depletion of IgG, as measured by IVIg or tracer IgG1 concentrations, was observed upon treatment with both Fc fragments containing the FcRn affinity enhancing MST-HN mutations. This depletion was dose dependent. A more pronounced depletion, as measured by IVIg serum concentration, was seen in response to Fc-G236A-MST-HN at either 5 mg/kg or 20 mg/kg compared to Fc-ΔG236-MST-HN **(****FIGs. 4B** and **4C****,** respectively).

To evaluate the PK profiles of the test items after a single IV administration, their levels in mouse serum were determined post-dose according to the bleeding scheme in **Table S7.** The obtained values were plotted over time **(****FIGs. 5A-5C****).** No difference in PK was seen across groups. PK does not seem to be affected by differences in FcyR binding of the Fc-MST-HN variants. ADA were detected in all mice beginning on d7 (data not shown).

In conclusion, this experiment suggests that FcyRIIa binding can enhance IgG depletion by Fc-MST-HN variants.

The invention is not to be limited in scope by the specific embodiments described herein. Indeed, various modifications of the invention in addition to those described will become apparent to those skilled in the art from the foregoing description and accompanying figures. Such modifications are intended to fall within the scope of the appended claims.

**The invention can also be understood with reference to the following numbered clauses:**
1. An FcRn antagonist comprising a variant IgG Fc region, wherein the variant IgG Fc region comprises or consists of a first Fc domain and a second Fc domain which form a dimer, wherein:
   (a) at least one of the first Fc domain and the second Fc domain comprise amino acids K, F, and Y at EU positions 433, 434, and 436, respectively; and
   (b) at least one of the first Fc domain and the second Fc domain comprise:
      (i) amino acid A at EU position 236; and/or
      (ii) amino acids D, D, D, D, G, and R, at EU positions 233, 237, 238, 268, 271, and 330, respectively.
2. An FcRn antagonist comprising a variant IgG Fc region, wherein the variant IgG Fc region comprises or consists of a first Fc domain and a second Fc domain which form a dimer, wherein:
   (a) at least one of the first Fc domain and the second Fc domain comprise amino acids Y, T, E, K, F, and Y at EU positions 252, 254, 256, 433, 434, and 436, respectively; and
   (b) at least one of the first Fc domain and the second Fc domain comprise:
      (i) amino acid A at EU position 236; and/or
      (ii) amino acids D, D, D, D, G, and R, at EU positions 233, 237, 238, 268, 271, and 330, respectively.
3. The FcRn antagonist of clause 2, wherein:
   (a) the first Fc domain comprises amino acids Y, T, E, K, F, and Y at EU positions 252, 254, 256, 433, 434, and 436, respectively, and the second Fc domain comprises amino acid A at EU position 236; or
   (b) the first Fc domain comprises amino acids A, Y, T, E, K, F, and Y at EU positions 236, 252, 254, 256, 433, 434, and 436, respectively, and the second Fc domain comprises amino acid A at EU position 236.
4. The FcRn antagonist of clause 3, wherein the second Fc domain further comprises amino acids K, F, and Y at EU positions 433, 434, and 436, respectively.
5. The FcRn antagonist of any one of clauses 1-4, wherein both the first Fc domain and the second Fc domain comprise amino acids Y, T, E, K, F, and Y at EU positions 252, 254, 256, 433, 434, and 436, respectively.
6. The FcRn antagonist of any one of clauses1-5, wherein both the first Fc domain and the second Fc domain comprise amino acid A at EU position 236.
7. The FcRn antagonist of any one of clauses 1-6, wherein the FcRn antagonist comprises two, three, or four FcRn binding regions.
8. The FcRn antagonist of any one of clauses 1-7, wherein the variant IgG Fc region binds to FcRn with a higher affinity at pH 6.0 as compared to a corresponding wild-type IgG Fc region.
9. The FcRn antagonist of any one of clauses 1-8, wherein the variant IgG Fc region binds to FcRn with a higher affinity at pH 7.4 as compared to a corresponding wild-type IgG Fc region.
10. The FcRn antagonist of any one of clauses 1-9, wherein the variant IgG Fc region binds to FcyRIIa with a higher affinity at pH 6.0 and/or at pH 7.4 as compared to a corresponding wild-type IgG Fc region.
11. The FcRn antagonist of any one of clauses 1-10, wherein the first Fc domain and/or the second Fc domain is an IgG1 Fc domain.
12. The FcRn antagonist of any one of clauses 1-10, wherein the first Fc domain and/or the second Fc domain is a human IgG Fc domain.
13. The FcRn antagonist of any one of clauses 1-12, wherein the first Fc domain and/or the second Fc domain is a human IgG1 Fc domain.
14. The FcRn antagonist of any one of clauses 1-11, wherein both the first Fc domain and the second Fc domain are IgG1 Fc domains.
15. The FcRn antagonist of any one of clauses 1-12, wherein both the first Fc domain and the second Fc domain are human IgG Fc domains.
16. The FcRn antagonist of any one of clauses 1-15, wherein both the first Fc domain and the second Fc domain are human IgG1 Fc domains.
17. The FcRn antagonist of any one of clauses 1-16, wherein the first Fc domain and/or the second Fc domain comprise or consist of an amino acid sequence independently selected from an amino acid sequence set forth in SEQ ID NOs: 7-9.
18. The FcRn antagonist of clause 17, wherein the first Fc domain and/or the second Fc domain comprise or consist of the amino acid sequence set forth in SEQ ID NO: 8.
19. The FcRn antagonist of any one of clauses 1-16, wherein both the first Fc domain and the second Fc domain comprise or consist of an amino acid sequence independently selected from an amino acid sequence set forth in SEQ ID NOs: 7-9.
20. The FcRn antagonist of clause 19, wherein both the first Fc domain and the second Fc domain comprise or consist of the amino acid sequence set forth in SEQ ID NO: 8.
21. The FcRn antagonist of any one of clauses 1-16, wherein the first Fc domain and/or the second Fc domain comprise or consist of an amino acid sequence independently selected from an amino acid sequence set forth in SEQ ID NOs: 13-15.
22. The FcRn antagonist of clause 21, wherein the first Fc domain and/or the second Fc domain comprise or consist of the amino acid sequence set forth in SEQ ID NO: 14.
23. The FcRn antagonist of any one of clauses 1-16, wherein both the first Fc domain and the second Fc domain comprise or consist of an amino acid sequence independently selected from an amino acid sequence set forth in SEQ ID NOs: 13-15.
24. The FcRn antagonist of clause 23, wherein both the first Fc domain and the second Fc domain comprise or consist of the amino acid sequence set forth in SEQ ID NO: 14.
25. The FcRn antagonist of any one of clauses 1-24, wherein the variant IgG Fc region is afucosylated.
26. A polypeptide comprising an Fc domain comprising amino acids A, Y, T, E, K, F, and Y at EU positions 236, 252, 254, 256, 433, 434, and 436, respectively.
27. The polypeptide of clause 26, comprising or consisting of an amino acid sequence selected from an amino acid sequence set forth in SEQ ID NOs: 7-9.
28. A polypeptide comprising an Fc domain comprising amino acids D, D, D, Y, T, E, D, G, R, K, F, and Y at EU positions 233, 237, 238, 252, 254, 256, 268, 271, 330, 433, 434, and 436, respectively.
29. The polypeptide of clause 28, comprising or consisting of an amino acid sequence selected from an amino acid sequence set forth in SEQ ID NOs: 13-15.
30. The polypeptide of any one of clauses 26-29, wherein the polypeptide is afucosylated.
31. A polynucleotide or polynucleotides encoding the FcRn antagonist of any one of clauses 1-25 or the polypeptide of any one of clauses 26-30.
32. An expression vector comprising the polynucleotide or polynucleotides of clause 31.
33. A host cell comprising the polynucleotide or polynucleotides of clause 31, or the expression vector of clause 32.
34. A method for producing an FcRn antagonist, comprising culturing the host cell of clause 33 under conditions which permit the expression of the FcRn antagonist.
35. A pharmaceutical composition comprising an FcRn antagonist of any one of clauses 1-25 or a polypeptide of any one of clauses 26-30, and at least one pharmaceutically acceptable carrier.
36. An FcRn antagonist of any one of clauses 1-25, a polypeptide of any one of clauses 26-30, or a pharmaceutical composition of clause 35 for use as a medicament.
37. A method of reducing serum levels of IgG in a subject comprising administering to the subject an effective amount of an FcRn antagonist of any one of clauses 1-25, a polypeptide of any one of clauses 26-30, or a pharmaceutical composition of clause 35.
38. A method of enhancing intracellular uptake of IgG in myeloid cells in a subject comprising administering to the subject an effective amount of an FcRn antagonist of any one of clauses 1-25, a polypeptide of any one of clauses 26-30, or a pharmaceutical composition of clause 35.
39. A method of treating an antibody-mediated disorder in a subject comprising administering to the subject an effective amount of an FcRn antagonist of any one of clauses 1-25, a polypeptide of any one of clauses 26-30, or a pharmaceutical composition of clause 35.
40. The method of clause 39, wherein the antibody-mediated disorder is an IgG-mediated disorder.
41. The method of clause 39 or 40, wherein the antibody-mediated disorder is an autoimmune disease.
42. The method of any one of clauses 37-41, wherein the FcRn antagonist or polypeptide is administered to the subject simultaneously or sequentially with an additional therapeutic agent.
43. An FcRn antagonist of any one of clauses 1-25, a polypeptide of any one of clauses 26-30, or a pharmaceutical composition of clause 35, for use in reducing serum levels of IgG, enhancing intracellular uptake of IgG in myeloid cells, or treating an antibody-mediated disorder, in a subject.
44. An FcRn antagonist of any one of clauses 1-25, or a polypeptide of any one of clauses 26-30, for the manufacture of a medicament for reducing serum levels of IgG, enhancing intracellular uptake of IgG in myeloid cells, or treating an antibody-mediated disorder, in a subject.
45. Use of an FcRn antagonist of any one of clauses 1-25, a polypeptide of any one of clauses 26-30, or a pharmaceutical composition of clause 35, for the manufacture of a medicament for reducing serum levels of IgG, enhancing intracellular uptake of IgG in myeloid cells, or treating an antibody-mediated disorder, in a subject.

## Claims

1. An FcRn antagonist comprising a variant IgG Fc region, wherein the variant IgG Fc region comprises or consists of a first Fc domain and a second Fc domain which form a dimer, wherein:
(a) at least one of the first Fc domain and the second Fc domain comprise amino acids K, F, and Y at EU positions 433, 434, and 436, respectively; and
(b) at least one of the first Fc domain and the second Fc domain comprise:
(i) amino acid A at EU position 236; and/or
(ii) amino acids D, D, D, D, G, and R, at EU positions 233, 237, 238, 268, 271, and 330, respectively.

2. The FcRn antagonist of claim 1, wherein at least one of the first Fc domain and the second Fc domain comprise amino acids Y, T, E, K, F, and Y at EU positions 252, 254, 256, 433, 434, and 436, respectively.

3. The FcRn antagonist of claim 2, wherein:
(a) the first Fc domain comprises amino acids Y, T, E, K, F, and Y at EU positions 252, 254, 256, 433, 434, and 436, respectively, and the second Fc domain comprises amino acid A at EU position 236, optionally wherein the second Fc domain further comprises amino acids K, F, and Y at EU positions 433, 434, and 436, respectively; or
(b) the first Fc domain comprises amino acids A, Y, T, E, K, F, and Y at EU positions 236, 252, 254, 256, 433, 434, and 436, respectively, and the second Fc domain comprises amino acid A at EU position 236, optionally wherein the second Fc domain further comprises amino acids K, F, and Y at EU positions 433, 434, and 436, respectively.

4. The FcRn antagonist of any one of claims 1-3, wherein both the first Fc domain and the second Fc domain comprise amino acids Y, T, E, K, F, and Y at EU positions 252, 254, 256, 433, 434, and 436, respectively; and/or
wherein both the first Fc domain and the second Fc domain comprise amino acid A at EU position 236.

5. The FcRn antagonist of any one of claims 1-4, wherein the FcRn antagonist comprises two, three, or four FcRn binding regions.

6. The FcRn antagonist of any one of claims 1-5, wherein the variant IgG Fc region binds to:
(a) FcRn with a higher affinity at pH 6.0 as compared to a corresponding wild-type IgG Fc region; and/or
(b) FcRn with a higher affinity at pH 7.4 as compared to a corresponding wild-type IgG Fc region; and/or
(c) FcyRIIa with a higher affinity at pH 6.0 and/or at pH 7.4 as compared to a corresponding wild-type IgG Fc region.

7. The FcRn antagonist of any one of claims 1-6, wherein:
(a) the first Fc domain and/or the second Fc domain is an IgG1 Fc domain; or
(b) the first Fc domain and/or the second Fc domain is a human IgG Fc domain, optionally a human IgG1 Fc domain.

8. The FcRn antagonist of any one of claims 1-7, wherein the first Fc domain and/or the second Fc domain comprise or consist of:
(a) an amino acid sequence independently selected from an amino acid sequence set forth in SEQ ID NOs: 7-9, preferably the amino acid sequence set forth in SEQ ID NO: 8; or
(b) an amino acid sequence independently selected from an amino acid sequence set forth in SEQ ID NOs: 13-15, preferably the amino acid sequence set forth in SEQ ID NO: 14.

9. A polypeptide comprising:
(a) an Fc domain comprising amino acids A, Y, T, E, K, F, and Y at EU positions 236, 252, 254, 256, 433, 434, and 436, respectively, optionally wherein the polypeptide comprises or consists of an amino acid sequence selected from an amino acid sequence set forth in SEQ ID NOs: 7-9; or
(b) an Fc domain comprising amino acids D, D, D, Y, T, E, D, G, R, K, F, and Y at EU positions 233, 237, 238, 252, 254, 256, 268, 271, 330, 433, 434, and 436, respectively, optionally wherein the polypeptide comprises or consists of an amino acid sequence selected from an amino acid sequence set forth in SEQ ID NOs: 13-15.

10. The FcRn antagonist of any one of claims 1-8 or the polypeptide of claim 9 wherein the variant IgG Fc region or polypeptide is afucosylated.

11. A polynucleotide or polynucleotides encoding the FcRn antagonist of any one of claims 1-8 or 10 or the polypeptide of claim 9 or 10.

12. An expression vector comprising the polynucleotide or polynucleotides of claim 11.

13. A host cell comprising the polynucleotide or polynucleotides of claim 11, or the expression vector of claim 12.

14. A method for producing an FcRn antagonist, comprising culturing the host cell of claim 13 under conditions which permit the expression of the FcRn antagonist.

15. A pharmaceutical composition comprising an FcRn antagonist of any one of claims 1-8 or 10 or a polypeptide of claim 9 or 10, and at least one pharmaceutically acceptable carrier.

16. An FcRn antagonist of any one of claims 1-8 or 10, a polypeptide of claim 9 or 10, or a pharmaceutical composition of claim 15 for use as a medicament.

17. An FcRn antagonist of any one of claims 1-8 or 10, a polypeptide of claim 9 or 10, or a pharmaceutical composition of claim 15, for use in reducing serum levels of IgG, enhancing intracellular uptake of IgG in myeloid cells, or treating an antibody-mediated disorder, in a subject.

18. The FcRn antagonist, polypeptide or pharmaceutical composition for use according to claim 17, wherein the antibody-mediated disorder is an IgG-mediated disorder; and/or wherein the antibody-mediated disorder is an autoimmune disease.

19. The FcRn antagonist, polypeptide or pharmaceutical composition for use according to claim 17 or 18, wherein the FcRn antagonist, polypeptide or pharmaceutical composition is administered to the subject simultaneously or sequentially with an additional therapeutic agent.
